(19) Europäisches Patentamt
European Patent Office
Office européen des brevets

(11) **EP 3 396 319 B1**

(12) **EUROPEAN PATENT SPECIFICATION**

(45) Date of publication and mention
of the grant of the patent:
**04.08.2021 Bulletin 2021/31**

(51) Int Cl.:
***G01C 21/26*** *(2006.01)*     ***G01C 21/16*** *(2006.01)*
***A61B 5/00*** *(2006.01)*     ***A61B 5/11*** *(2006.01)*
***G01C 22/00*** *(2006.01)*

(21) Application number: **15911358.8**

(22) Date of filing: **24.12.2015**

(86) International application number:
**PCT/JP2015/086130**

(87) International publication number:
**WO 2017/109920 (29.06.2017 Gazette 2017/26)**

(54) **INFORMATION PROCESSING SYSTEM, INFORMATION PROCESSING PROGRAM, AND INFORMATION PROCESSING METHOD**

INFORMATIONSVERARBEITUNGSSYSTEM, INFORMATIONSVERARBEITUNGSPROGRAMM UND INFORMATIONSVERARBEITUNGSVERFAHREN

SYSTÈME DE TRAITEMENT D'INFORMATIONS, PROGRAMME DE TRAITEMENT D'INFORMATIONS ET PROCÉDÉ DE TRAITEMENT D'INFORMATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**31.10.2018 Bulletin 2018/44**

(73) Proprietor: **Fujitsu Limited**
**Kawasaki-shi, Kanagawa 211-8588 (JP)**

(72) Inventors:
• **HOTTA, Shinji**
**Kawasaki-shi**
**Kanagawa 211-8588 (JP)**
• **INOMATA, Akihiro**
**Hayes, Middlesex UB4 8FE (GB)**

(74) Representative: **Haseltine Lake Kempner LLP**
**Cheapside House**
**138 Cheapside**
**London**
**EC2V 6BJ (GB)**

(56) References cited:
**WO-A1-2014/118767     JP-A- 2000 325 329**
**JP-A- 2008 220 635     JP-A- 2013 196 442**

• **None**

**Description**

[Technical Field]

The present invention relates to an information processing system and the like.

[Background Art]

Reference may be made to WO 2014/118767 A1, which relates to: a method for automatic detection of types of locomotion, including acquiring a motion signal from a sensor attached to a subject during a period of time when the subject is in motion, extracting one or more features from the motion signal, inputting the one or more features to a locomotion recognition unit, and having the locomotion recognition unit produce an output indicating a likelihood that the subject was moving using a specific type of locomotion during the period of time; and to a system for automatic classification of different types of locomotion, including a locomotion classification module configured to accept input of a motion signal from a motion sensor and to produce output including an indication of a locomotion classification based, at least in part, on the motion signal.

A technique of detecting gait initiation and gait termination by using sensor on both legs has conventionally been known. For example, there is a technique of detecting a sign of gait initiation based on angles of a back and a knee and then detecting toe-off based on a foot pressure on a shoe sole, thereby detecting gait initiation (for example, refer to Non Patent Document 1).

Furthermore, there is a technique in which a mobile terminal detects, when a walker holding the mobile terminal in his/her hand changes the direction of his/her steps, the direction change (for example, refer to Patent Document 1). In the technique, a mobile terminal including an acceleration sensor and a GPS unit determines that a walker has changed the direction of his/her steps when the following conditions are met. The conditions specifically are that a first azimuth difference between a first traveling direction at a first time point and a third traveling direction at a third time point is equal to or larger than an angle threshold, and that a second azimuth difference between a second traveling direction at a second time point and a fourth traveling direction at a fourth time point is equal to or larger than the angle threshold.

[Citation List]

[Patent Citation]

Patent Document 1: Japanese Laid-open Patent
Publication no. 2009-75036
Patent Document 2: Japanese Laid-open Patent Publication No. 10-113343
Patent Document 3: Japanese Laid-open Patent Publication No. 2008-027429
Patent Document 4: Japanese Laid-open Patent Publication No. 2009-150711
Patent Document 5: Japanese Laid-open Patent Publication No. 2010-223829

[Non Patent Citation]

Non Patent Document 1: "Automated detection of gait initiation and termination using wearable sensors Medical Engineering & Physics, Vol. 35, Issue 12, pp. 1713-1720"

[Summary of Invention]

[Technical Problem]

However, there has been a problem in conventional techniques that a feature amount of a direction change motion cannot be extracted highly accurately only with sensors on both legs.

For example, in the technique in which a mobile terminal detects a direction change, the mobile terminal can detect a section of the direction change based on changes in the azimuth difference by using a sensor at a position at which the mobile terminal is held in a hand, but cannot detect a feature amount of individual direction change motions with high accuracy. This is because the sensor positioned at a position at which the terminal is held in a hand cannot detect individual step actions when a direction change is made with significantly small steps.

If the technique of detecting a gait initiation with sensors on both legs and the technique in which a mobile terminal detects a direction change are combined, a feature amount of a direction change motion can be extracted; however, sensors are necessary to be worn not only on both legs but also on an upper body.

In one aspect, it is an object to extract a feature amount of a direction change motion highly accurately only with sensors on both legs.

[Solution to Problem]

The present invention is defined by the independent claims, to which reference should now be made. Specific embodiments are defined in the dependent claims.

[Advantageous Effects of Invention]

According to one embodiment, a feature amount of a direction change motion can be extracted highly accurately only with sensors on both legs.

[Brief Description of Drawings]

FIG. 1 is a functional block diagram illustrating a configuration of an information processing system according to an embodiment.
FIG. 2A is a diagram for explaining directions of axes of a sensor device.
FIG. 2B is a diagram illustrating one example of wearing of the sensor devices.
FIG. 3 is a diagram for explaining direction-change section determination according to the embodiment.
FIG. 4 is a diagram illustrating one example of peak detection.
FIG. 5 is a diagram for explaining gait section determination according to the embodiment.
FIG. 6 is a flowchart of entire information processing according to the embodiment.
FIG. 7A is a flowchart of direction-change-section extraction processing according to the embodiment.
FIG. 7B is a flowchart of the direction-change-section extraction processing according to the embodiment.
FIG. 7C is a flowchart of the direction-change-section extraction processing according to the embodiment.
FIG. 8 is a flowchart of direction-change feature-amount-extraction processing according to the embodiment.
FIG. 9A is a flowchart of transition -gait-section extraction processing according to the embodiment.
FIG. 9B is a flowchart of the transition -gait-section extraction processing according to the embodiment.
FIG. 9C is a flowchart of the transition -gait-section extraction processing according to the embodiment.
FIG. 10 is a flowchart of transition-gait-feature extraction processing according to the embodiment.
FIG. 11A illustrates a specific example of motion-candidate extraction processing according to the embodiment.
FIG. 11B illustrates a specific example of the motion-candidate extraction processing according to the embodiment.
FIG. 11C illustrates a specific example of the motion-candidate extraction processing according to the embodiment.
FIG. 11D illustrates a specific example of the motion-candidate extraction processing according to the embodiment.
FIG. 11E illustrates a specific example of the motion-candidate extraction processing according to the embodiment.
FIG. 12A illustrates a specific example of direction-change-section determination processing according to the embodiment.
FIG. 12B illustrates a specific example of the direction-change-section determination processing according to the embodiment.
FIG. 12C illustrates a specific example of the direction-change-section determination processing according to the embodiment.
FIG. 12D illustrates a specific example of the direction-change-section determination processing according to the embodiment.
FIG. 13A illustrates a specific example of direction-change-feature extraction processing according to the embodiment.
FIG. 13B illustrates a specific example of the direction-change-feature extraction processing according to the embodiment.
FIG. 13C illustrates a specific example of the direction-change-feature extraction processing according to the embodiment.
FIG. 14A illustrates a specific example of gait section identification processing according to the embodiment.
FIG. 14B illustrates a specific example of the gait section identification processing according to the embodiment.
FIG. 14C illustrates a specific example of the gait section identification processing according to the embodiment.
FIG. 14D illustrates a specific example of the gait section identification processing according to the embodiment.

FIG. 15 illustrates a specific example of gait-feature extraction processing according to the embodiment.
FIG. 16 illustrates another specific example of the gait-feature extraction processing according to the embodiment.
FIG. 17 illustrates a specific example of feature-amount storage processing according to the embodiment.
FIG. 18 illustrates a specific example of feature-amount visualization processing according to the embodiment.
FIG. 19 illustrates one example of a computer that executes an information processing program.

[Embodiments for Carrying Out the Invention]

Embodiments of an information processing system, an information processing program, and an information processing method disclosed in the present application are explained in detail below based on the accompanying drawings. The embodiments are not intended to limit the present invention.

Embodiment

[Configuration of Information Processing System]

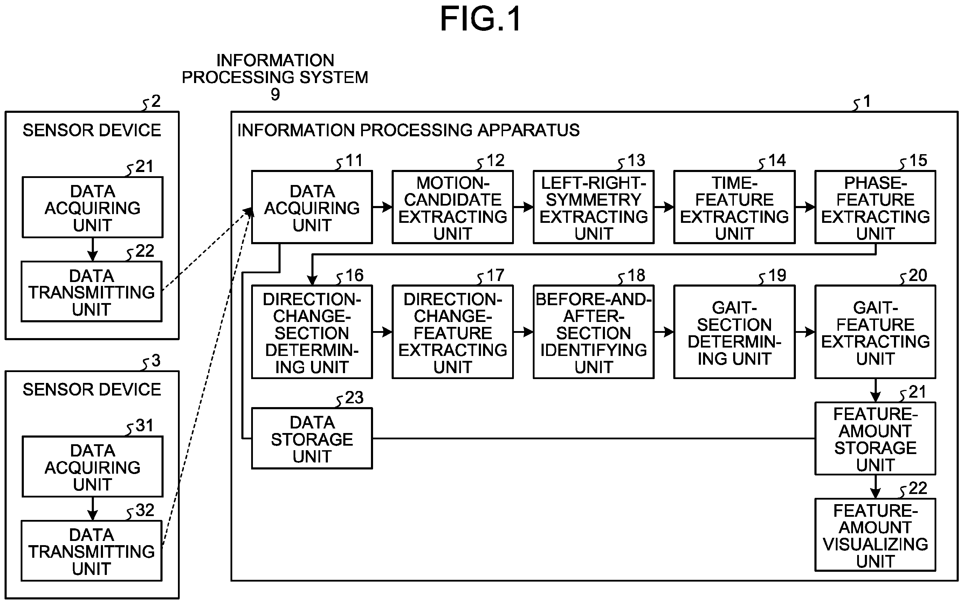

FIG. 1 is a functional block diagram illustrating a configuration of an information processing system according to an embodiment. As illustrated in FIG. 1, an information processing system 9 includes an information processing apparatus 1, and sensor devices 2, 3. The information processing apparatus 1 is connected to each of the sensor device 2 and the sensor device 3.

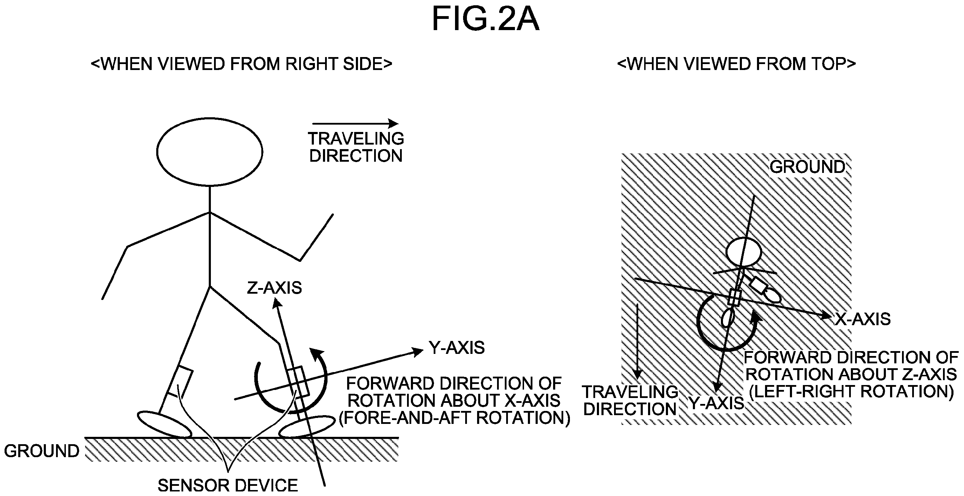

The sensor device 2 is an angular velocity sensor (gyro sensor) around three axes that are perpendicular to each other, and is worn, for example, on a left leg of a subject. Axis directions of the sensor device 2 are explained referring to FIG. 2A.

FIG. 2A is a diagram for explaining directions of axes of a sensor device. As illustrated in a diagram, "WHEN VIEWED FROM TOP" in FIG. 2A, an angular velocity about a Z-axis is to be a displacement value along with rotation in a traverse (left-right) direction in walking of the subject. That is, it is a rotation amount in a left-right direction relative to a worn posture of the sensor device at a predetermined time point and, for example, a rotation amount in a rightward direction is plus (forward direction), and a rotation amount in a leftward direction is minus. As illustrated in a diagram, "WHEN VIEWED FROM RIGHT SIDE" in FIG. 2A, an angular velocity about an X-axis is to be a displacement value along with rotation in a fore-and-aft direction in walking of the subject. That is, it is a rotation amount in a fore-and-aft direction relative to the worn posture of the sensor device 2 at a predetermined time point, and a rotation amount in a backward direction (lift-up direction) is plus (forward direction), and a rotation amount in a frontward direction (forward-leaning direction) is minus. An angular velocity about a Y-axis is to be a displacement value along with rotation in a body axis direction in walking of the subject. That is, it is a rotation amount in the left-right direction relative to the worn posture of the sensor device at a predetermined time point, and a rotation amount in a rightward direction is plus, and a rotation amount in a leftward direction is minus. The sensor device 2 can include an acceleration sensor that detects acceleration in three axes perpendicular to each other. Moreover, the sensor device 2 includes a gyro sensor. In the following, explanation is given assuming that the sensor device 2 is a gyro sensor.

The sensor device 2 includes a data acquiring unit 21 and a data transmitting unit 22. The data acquiring unit 21 acquires gyro data. The data transmitting unit 22 transmits the gyro data acquired by the data acquiring unit 21 to the information processing apparatus 1.

The sensor device 3 is the same device as the sensor device 2, and is worn, for example, on a right leg of the subject. As the function thereof is the same as that of the sensor device 2, explanation thereof is omitted.

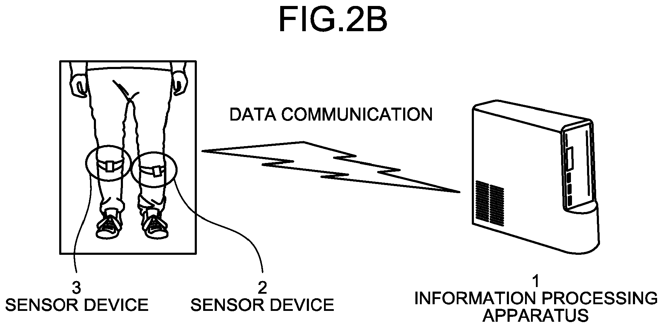

An example of wearing of the sensor devices 2, 3 is explained, referring to FIG. 2B. FIG. 2B is a diagram illustrating one example of wearing of the sensor devices. As illustrated in FIG. 2B, the sensor devices 2, 3 are worn on both legs of a subject, respectively. Specifically, the sensor device 2 is worn on the left leg of the subject, and the sensor device 3 is worn on the right leg of the subject. The sensor devices 2, 3 communicate with the information processing apparatus 1, and transmit various kinds of data to the information processing apparatus 1 according to a direction change motion and a gait motion of the subject.

Referring back to FIG. 1, the information processing apparatus 1 extracts feature amounts of the direction change motion and the gait motion of the subject by using only data from the sensor devices 2, 3 worn on the legs of the subject. The information processing apparatus 1 includes a data acquiring unit 11, a motion-candidate extracting unit 12, a left-right-symmetry extracting unit 13, a time-feature extracting unit 14, a phase-feature extracting unit 15, a direction-change-section determining unit 16, and a direction-change-feature extracting unit 17. In addition, the information processing apparatus 1 includes a before-and-after-section identifying unit 18, a gait-section determining unit 19, a gait-feature extracting unit 20, a feature-amount storage unit 21, a feature-amount visualizing unit 22, and a data storage unit 23. The sensor devices 2, 3 are one example of a first motion sensor and a second motion sensor, respectively. The information processing apparatus 1 is one example of an information processing unit.

The data acquiring unit 11 receives various kinds of data that are transmitted respectively from the sensor devices 2, 3, and accumulates the received various kinds of data in the data storage unit 23. For example, the data acquiring unit 11 receives 60 samples each of the various kinds of data in one second, and accumulates the received various kinds of data. The various kinds of data include a rotation amount in a traverse (left-right) direction, a rotation amount in a fore-and-aft direction, and a rotation amount of a body axis. The various kinds of data are accumulated in the data storage unit 23 every each time point.

Having acquired a start instruction to extract a feature of a direction change, the data acquiring unit 11 reads various kinds of data of a corresponding subject from the data storage unit 23 based on the acquired instruction. The data acquiring unit 11 outputs the read various kinds of data to the motion-candidate extracting unit 12.

The motion candidate extracting unit 12 extracts a portion at which a rotation amount in a traverse direction is larger than a first threshold, as a motion candidate. For example, the motion-candidate extracting unit 12 detects a peak from an angular velocity toward a traverse direction from among the various kinds of data that are output from the data acquiring unit 11. The peak detection is performed, applying a lowpass filter, such as a Butterworth filter, to the angler velocity data. The motion-candidate extracting unit 12 extracts a portion around this peak in one unit as a motion candidate of the direction change when the rotation amount toward the traverse direction around this peak is larger than the first threshold. When peaks of rotation in the same traverse direction appear successively in the same leg within predetermined time, the motion-candidate extracting unit 12 can group these peaks and detect as one peak. This is because when a human takes a step, motion is complicated in rotation in the traverse direction compared to movement to a forward direction and peaks are likely to appear successively. Thus, the motion-candidate extracting unit 12 can extract one motion candidate of the direction change per step.

The left-right-symmetry extracting unit 13 compares the motion candidate that is extracted by the motion-candidate extracting unit 12 with a next previous motion candidate, and extracts whether an actin candidate of the left leg and a motion candidate of the right leg appear alternately.

The time-feature extracting unit 14 extracts a time interval between the motion candidate that is extracted by the motion-candidate extracting unit 12 and a next previous motion candidate.

The phase-feature extracting unit 15 extracts whether rotation in the traverse direction of the motion candidate that is extracted by the motion-candidate extracting unit 12 is in the same direction (same phase) as a next previous motion candidate.

The direction-change-section determining unit 16 determines a section in which the feature extracted by the time-feature extracting unit 14, the feature extracted by the left-right-symmetry extracting unit 13, and the feature extracted by the phase-feature extracting unit 15 satisfy a predetermined condition as a direction change section. Specifically, the direction-change-section determining unit 16 determines a section of a motion candidate in which the rotations in the traverse direction are in the same direction, and the left leg and the right leg rotate alternately, and that appears chronically successively, as the direction change motion, out of plural motion candidates extracted by the motion-candidate extracting unit 12.

Note that the method of determining the direction change section by the direction-change-section determining unit 16 is not limited to the method by condition-based determination described above. The direction-change-section determining unit 16 can determine the direction change section by probability by using the hidden Markov model (HMM) or the like. The method of determining the direction change section by using the hidden Markov model (HMM) is explained later.

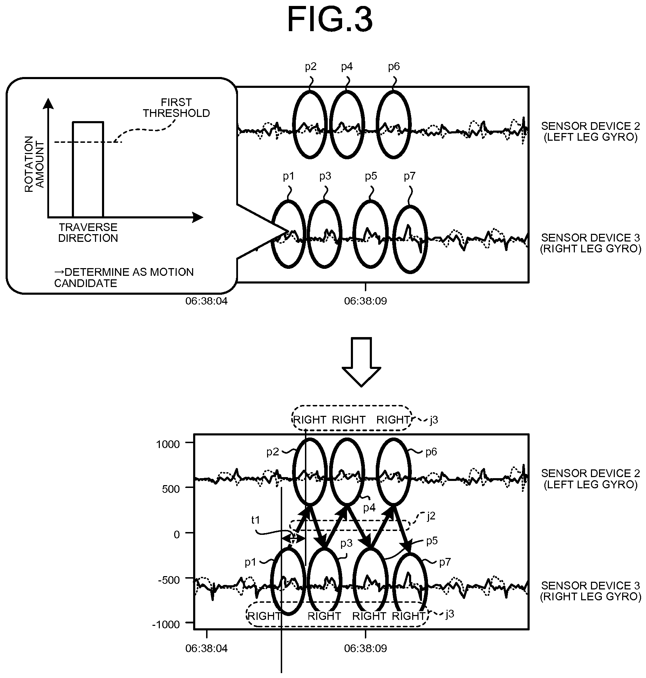

Direction-change section determination according to the embodiment is explained, referring to FIG. 3. FIG. 3 is a diagram for explaining the direction-change section determination according to the embodiment. As expressed by solid lines in an upper diagram and a lower diagram in FIG. 3, the angular velocity in the traverse direction of the left leg transmitted from the sensor device 2 and the angular velocity in the traverse direction of the right leg transmitted from the sensor device 3 are indicated chronologically in graphs. Doted lines in the graphs indicate the angular velocity in the forward direction.

The motion-candidate extracting unit 12 applies a lowpass filter to a rotation amount in the traverse direction to detect a peak, and extracts, when a rotation amount in the traverse direction from a start of the peak until an end of the peak is larger than the first threshold, a portion around this peak in one unit as a motion candidate of the direction change. In this example, the rotation amount in the traverse direction of peaks p1 to p7 are larger than the first threshold. Therefore, the motion-candidate extracting unit 12 extracts the respective peaks p1 to p7 as motion candidates of the direction change.

The time-feature extracting unit 14 extracts a time interval between the motion candidate that is extracted by the motion-candidate extracting unit 12 and a next previous motion candidate. In this example, a time interval between the peaks p1 and p2 being motion candidates is extracted as t1, as an example. Similarly, a time interval between the peaks p2 and p3 being motion candidates is extracted. A time interval between the peaks p3 and p4 as motion candidates is extracted. A time interval between the peaks p4 and p5 being motion candidates is extracted. A time interval between the peaks p5 and p6 being motion candidates is extracted.

The left-right-symmetry extracting unit 13 compares the motion candidate that is extracted by the motion-candidate extracting unit 12 with a next previous motion candidate, and extracts whether a motion candidate of the left leg and a motion candidate of the right leg appear alternately. In this example, as the peak p2 is a motion candidate of the left leg and the next previous motion candidate, the peak p1 is a motion candidate of the right leg, alternate appearance is extracted. Similarly, as the peak p3 is a motion candidate of the right leg and the next previous motion candidate, the peak p2 is the motion candidate of the left leg, alternate appearance is extracted. As the peak p4 is a motion candidate of the left leg and the next previous motion candidate, the peak p3 is the motion candidate of the right leg, alternate appearance is extracted. As the peak p5 is a motion candidate of the right leg and the next previous motion candidate, the peak p4 is the motion candidate of the left leg, alternate appearance is extracted. As the peak p6 is a motion candidate of the left leg and the next previous motion candidate, the peak p5 is the motion candidate of the right leg, alternate appearance is extracted. As the peak p7 is a motion candidate of the left leg and the next previous motion candidate, the peak p6 is the motion candidate of the right leg, alternate appearance is extracted.

The phase-feature extracting unit 15 extracts whether rotation in the traverse direction of the motion candidate that is extracted by the motion-candidate extracting unit 12 is in the same direction (same phase) as that of the next previous motion candidate. In this example, as signs of the rotation amounts of the peaks p1 to p7 are all plus, it is extracted that all of them is in the same direction. Therefore, it is extracted that the peaks p1 to p7 are of the same direction (same phase).

The direction-change-section determining unit 16 determines a section in which the feature extracted by the time-feature extracting unit 14, the feature extracted by the left-right-symmetry extracting unit 13, and the feature extracted by the phase-feature extracting unit 15 satisfy a predetermined condition as a direction change section. Specifically, the direction-change-section determining unit 16 determines a section of a motion candidate in which the rotations in the traverse direction are in the same direction, and the left leg and the right leg rotate alternately, and that appears chronically successively, as the direction change motion, out of plural motion candidates extracted by the motion-candidate extracting unit 12. In this example, it is determined that the peaks p1 to p7 are motion candidates that appear chronically successively based on the feature extracted by the time-feature extracting unit 14. it is determined that the peaks p1 to p7 are motion candidates in which the left leg and the right leg rotate alternately (j2) based on the feature extracted by the left-right-symmetry extracting unit 13. It is determined that the peaks p1 to p7 are motion candidates of the same phase based on the feature extracted by the phase-feature extracting unit 15 (j3). Therefore, the section of the peaks p1 to p7 of the motion candidates is determined as the direction change section.

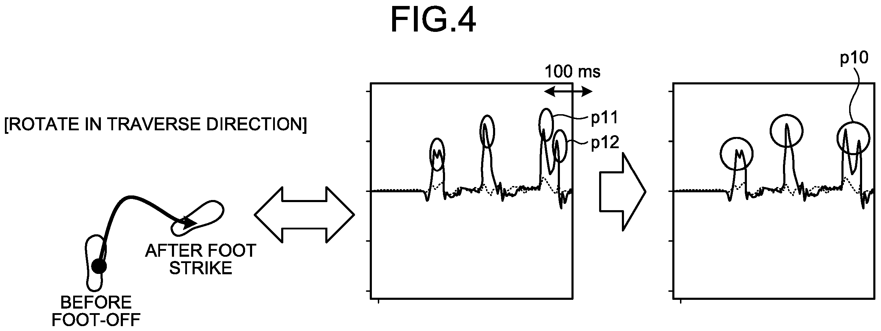

A method of detection a peak detected by the motion-candidate extracting unit 12 is explained, referring to FIG. 4. FIG. 4 is a diagram illustrating one example of peak detection. As illustrated in FIG. 4, when a human takes a step, motion is often complicated in rotation in the traverse direction compared to movement to a forward direction. When the motion is complicated in rotation in the traverse direction, multiple peaks appear successively. An example of a case in which successive multiple peaks occur is when a leg rotates just before landing in a period from a pre-foot-off to a post-foot-strike. The pre-foot-off is before a foot is taken off. The post-foot-strike is after a foot strikes on. In this example, it is assumed that multiple peaks p11, p12 occur when one leg is rotated just one step in the traverse direction.

In this case, the motion candidate extracting unit 12 groups, when peaks of rotation in the same traverse direction successively appear in the same leg within predetermined time, these peaks. In this example, the predetermined time is 100 ms. As the peak p11 and the peak p12 are of the same leg, and are rotations in the same traverse direction, and appear successively. Furthermore, an interval between the peak p11 and the peak p12 is smaller than 100 ms. Therefore, the motion-candidate extracting unit 12 groups the peak p11 and the peak p12 as a peak p10. Thus, the motion-candidate extracting unit 12 can extract one motion candidate of the direction change of one step. Note that multiple peaks that appear successively in predetermine time is referred to as "multiplex peaks", and grouping multiplex peaks is referred to as "chunking processing".

Referring back to FIG. 1, the direction-change-feature extracting unit 17 extracts a feature of a direction change motion that has been determined by the direction-change-section determining unit 16, using data stored in the data storage unit 23. For example, the direction-change-feature extracting unit 17 extracts features relating to (A) how fast a direction change was done, (B) how large it was rotated, (C) whether it could rotate large from a start, (D) whether a direction change could be done stably, (E) whether a still state occurred during a direction change, and (F) how asymmetric between a left leg and a right leg. As an example of (A), <1> a frequency of direction change motions during a direction change is extracted. <2> a maximum duration of a direction change motion during a direction change is extracted. <3> An average duration of a direction change motion during a direction change is extracted. As an example of (B), <4> a maximum rotation amount of a direction change motion during a direction change is extracted. <5> An average rotation amount of a direction change motion during a direction change is extracted. As an example of (C), <6> a rotation amount of a first direction change motion during a direction change is extracted. As an example of (D), <7> an interval between adjacent direction change motions during a direction change is extracted. <8> A lurch in a traverse direction right after foot strike is extracted. As an example of (E), <9> whether a still state is present, or a duration of a still state when a still state is present during a direction change is extracted. The features of a direction change motion that is extracted by the direction-change-feature extracting unit 17 are one example, and are not limited thereto.

The before-and-after-section identifying unit 18 identifies a peak, the rotation amount in the traverse direction is equal to or smaller than a first threshold and equal to or larger than a second threshold out of peaks that occur right after (or right before) a direction change section that has been determined by the direction-change-section determining unit 16. The second threshold is a threshold smaller than the first threshold. That is, right after (or right before) a direction change motion is determined, a direction change can be made while walking. Therefore, the before-and-after-section identifying unit 18 identifies peaks when a direction change is made while walking. Such a direction change that occurs before and after a direction change is referred to as "mild direction change". Thus, a length of a section in which the identified mild direction change continues is extracted as a feature amount, and the before-and-after-section identifying unit 18 can thereby determine a difficulty of a direction change. Moreover, the before-and-after-section identifying unit 18 can determine a lurch after a direction change by extracting whether a direction of a mild direction change in the traverse direction is an opposite direction to a direction change right before that.

The gait-section determining unit 19 identifies a gait section candidate between two direction change sections. For example, the gait-section determining unit 19 detects peaks from an angular velocity to a forward direction between two direction change sections, and identifies a peak, the rotation amount of which to the forward direction is equal to or larger than a threshold R1 out of the detected peaks. The gait-section determining unit 19 identifies a section in which the identified peaks successively occur in a short period of time as a gait section candidate. The gait-section determining unit 19 identifies a peak, the rotation amount to the forward direction of which is smaller than the threshold R1 and equal to or larger than the threshold R2 before and after the gait section candidate. The threshold R2 is a threshold smaller than the threshold R1. Such a section of peaks identified before and after a gait section candidate is referred to as "transition gait section". In other words, the "transition gait section" is a section of unstable walking until the walking is stabilized. The gait-section determining unit 19 determines the first peak in a transition gait section as the "first step", and determines the last peak as the "last step". A section including the transition gait section and the gait section candidate is referred to as "gait section" for convenience.

The method of determining the "gait section" is not limited thereto. The gait-section determining unit 19 can determine the gait section by using the hidden Markov model (HMM). The method of determining the "gait section" by using the hidden Markov model (HMM) is described later. Furthermore, as the "transition gait section" is an unstable section in terms of walking and a section of the mild direction change is an unstable section in terms of direction change, these sections can be determined in a duplicated manner. Moreover, the method of determining the transition gait section is not limited thereto. As another example, the gait-section determining unit 19 can determine a section from the first step until first several steps (for example, four steps) are completed as the transition gait section, out of a section that has been determined as the gait section. A method of determining the transition gait section by using a distribution of time intervals of peaks is described later.

The gait-feature extracting unit 20 extracts a feature of the transition gait section that has been identified by the gait-section determining unit 19 by using data stored in the data storage unit 23. For example, the gait-feature extracting unit 20 extracts features relating to (a) how fast it turned into stable walking, and (b) with how few lurch, it turned into stable walking. As an example of (a), time and the number of steps until it turned into stable walking are extracted. As an example of (b), a rotation amount in a traverse direction until it turned into stable walking is extracted.

The feature-amount storage unit 21 stores feature amounts in the direction change section and the transition gait section chronologically in the data storage unit 23. For example, the feature-amount storage unit 21 stores various kinds of feature amount of the direction change section extracted by the direction-change-feature extracting unit 17 and various kinds of feature amount of the transition gait section extracted by the gait-feature extracting unit 20 in order of occurrence in the data storage unit 23.

The feature-amount visualizing unit 22 visualizes various kinds of feature amount stored in the data storage unit 23 in chronological order. For example, when the subject is a patient, the feature-amount visualizing unit 22 visualizes statistical values of various kinds of feature amount per month for sections before and after relative to the day on which the patient is treated, to monitor the treatment progress. An example of the feature amount includes the number of times of direction change motion during a direction change.

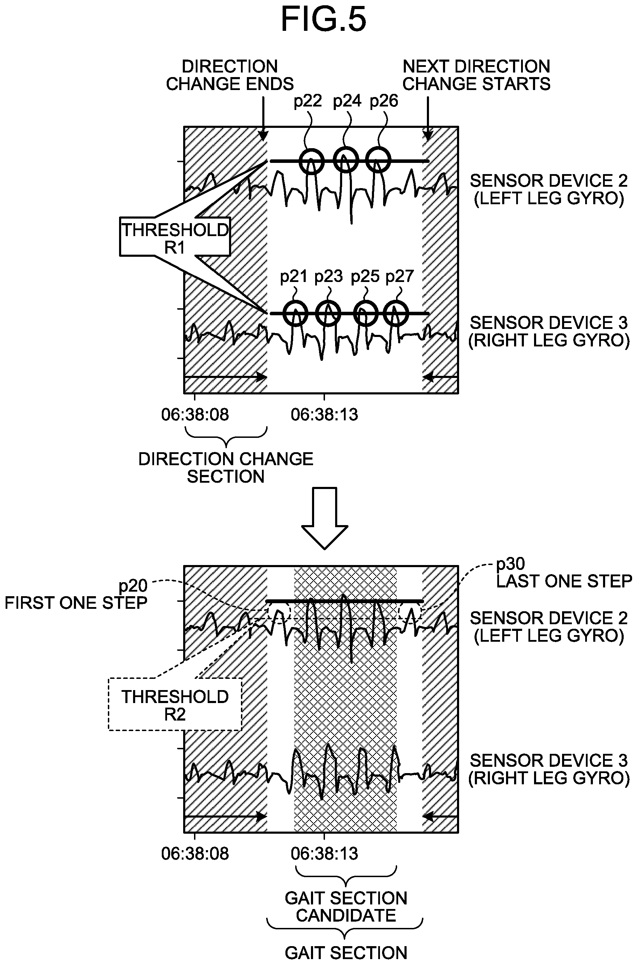

Gait section determination according to the embodiment is explained, referring to FIG. 5. FIG. 5 is a diagram for explaining the gait section determination according to the embodiment. As illustrated in upper and lower diagrams in FIG. 5, the angular velocity in the forward direction of the left leg transmitted from the sensor device 2 and the angular velocity in the forward direction of the right leg transmitted from the sensor device 3 are indicated chronologically in graphs per time.

The gait-section determining unit 19 applies a lowpass filter to the rotation amount in the forward direction between two direction change sections to detect peaks, and identifies a peak, the rotation amount in the forward direction of which is equal to or larger than the threshold R1. In this example, respective rotation amounts of peaks p21 to p27 are equal to or larger than the threshold R1. Therefore, the gait-section determining unit 19 identifies a section in which the peaks p21 to p27 successively occur in a short period of time as a gait section candidate.

The gait-section determining unit 19 identifies peaks before and after the gait section candidate, and identifies a peak, the rotation amount in the forward direction of which is smaller than the threshold R1 and equal to or larger than the threshold R2 out of the identified peaks. In this example, rotation amounts of peak p20 and p30 in the forward direction are smaller than the threshold R1 and equal to or larger than the threshold R2. Therefore, the gait-section determining unit 19 determines that the peak p20 before the gait section candidate as the first one step. The gait-section determining unit 19 determines the peak p30 after the gait section candidate as the last one step.

[Flowchart of Entire Information Processing]

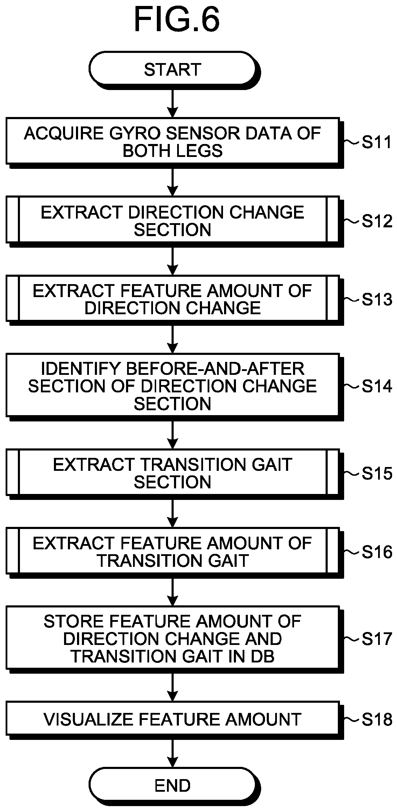

Next, a flowchart of entire information processing according to the embodiment is explained, referring to FIG. 6. FIG. 6 is a flowchart of entire information processing according to the embodiment. It is assumed that the data acquiring unit 11 has accepted a start instruction to extract a feature of a direction change for a specific subject. It is assumed that gyro sensor data from the sensor devices 2, 3 that are worn by the specific subject is stored in the data storage unit 23.
First, the data acquiring unit 11 acquires gyro sensor data corresponding to the specific subject from the data storage unit 23 (step S11). The direction-change-section determining unit 16 extracts a direction change section (step S12). A detailed flowchart is described later.
The direction-change-feature extracting unit 17 extracts a feature amount of the direction change (step S13). A detailed flowchart is described later.
The before-and-after-section identifying unit 18 identifies sections before and after the direction change section (step S14). For example, the before-and-after-section identifying unit 18 identifies a peak, the rotation amount in the traverse direction of which is equal to or smaller than the first threshold and equal to or larger than the second threshold out of peaks that occur right after the direction change section. That is, the before-and-after-section identifying unit 18 identifies a section of a mild change of direction (mild direction change) that occurs before and after the direction change section.
The gait-section determining unit 19 extracts a transition gait section (step S15). A detailed flowchart is described later.
The gait-feature extracting unit 20 extracts a feature amount of transition gait from the transition gait section (step S16). A detailed flow chart is described later.
The feature-amount storage unit 21 stores feature amounts of a direction change and a transition gait in a DB (database) (step S17). The feature-amount storage unit 21 stores various kinds of feature amount of the direction change section extracted by the direction-change-feature extracting unit 17 and various kinds of feature amount of the transition gait section extracted by the gait-feature extracting unit 20 in order of occurrence in the data storage unit 23.
The feature-amount visualizing unit 22 visualizes a feature amount (step S18). For example, the feature-amount visualizing unit 22 visualizes various kinds of feature amount stored in the data storage unit 21 in chronological order.

[Flowchart of Direction-Change-Section Extraction Processing]

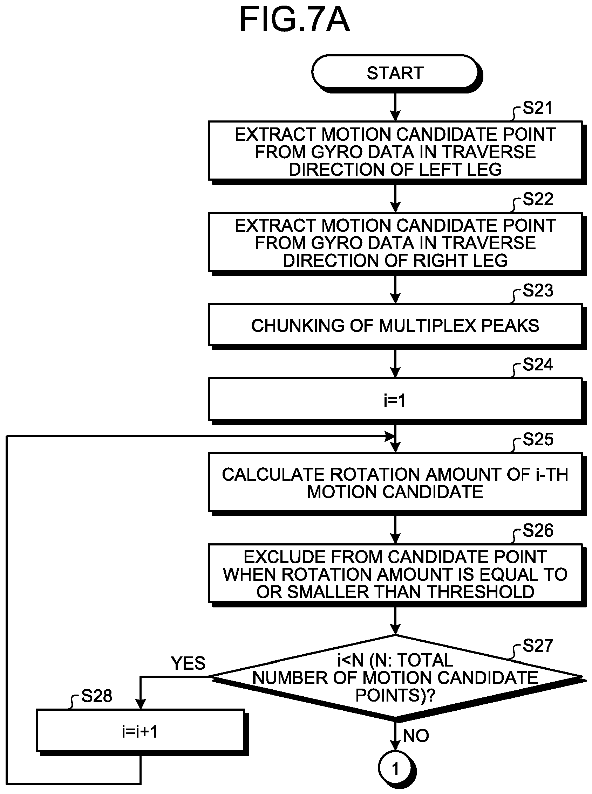

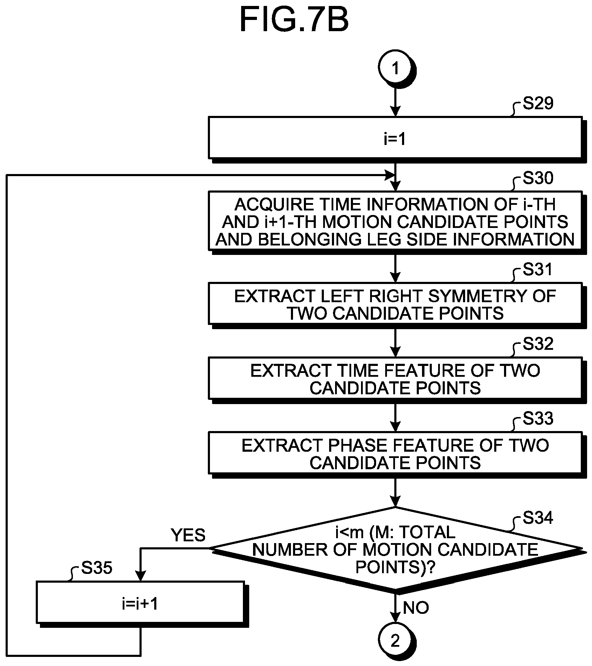

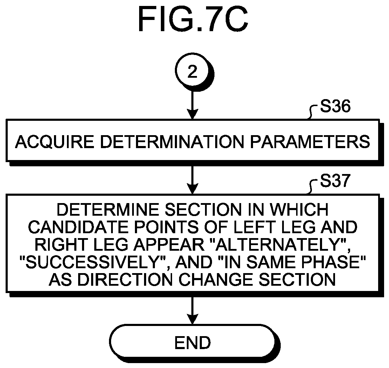

Next, a flowchart of direction-change-section extraction processing according to the embodiment is explained, referring to FIG. 7A to FIG. 7C. FIG. 7A to FIG. 7C are flowcharts of the direction-change-section extraction processing according to the embodiment.
As illustrated in FIG. 7A, the motion-candidate extracting unit 12 extracts a motion candidate point from traverse direction gyro data of the left leg (step S21). For example, the motion-candidate extracting unit 12 detects a peak from the rotation amount in the traverse direction of the left leg out of data stored in the data storage unit 23.
The motion-candidate extracting unit 12 extracts a motion candidate point from traverse direction gyro data of the right leg (step S22). For example, the motion-candidate extracting unit 12 detects a peak from the rotation amount in the traverse direction of the right leg out of data stored in the data storage unit 23.
The motion-candidate extracting unit 12 performs chunking processing of multiplex peaks (step S23). That is, the motion-candidate extracting unit 12 groups, when peaks of rotation in the same traverse direction appear successively in the same leg within predetermined time (for example, 100 ms), the peaks into one unit of peak.
The motion-candidate extracting unit 12 sets an initial value 1 to an index i (step S24). The motion-candidate extracting unit 12 calculates a rotation amount in the traverse direction at an i-th motion candidate point (step S25). The motion-candidate extracting unit 12 excludes the calculated rotation amount in the traverse direction when it is equal to or smaller than a threshold (step S26). The threshold herein is, for example, the first threshold described above.
The motion-candidate extracting unit 12 determines whether the index i is smaller than a total number N of motion candidate points (step S27). When the index i is smaller than the total number N of motion candidate points (step S27: YES), the motion-candidate extracting unit 12 adds 1 to the index i (step S28), and shifts to step S25 to process the next index i.
On the other hand, when the index i is equal to or larger than the total number N of motion candidate points (step S27: NO), the direction-change-section determining unit 16 sets the initial value 1 to the index i (step S29). The direction-change-section determining unit 16 acquires time information and information of a belonging leg at i-th and i+1-th motion candidate points (step S30).

The left-right-symmetry extracting unit 13 extracts left-right symmetry of two motion candidate points (step S31). That is, the left-right-symmetry extracting unit 13 extracts a feature indicating whether the left leg and the right leg alternately appear, for successive two motion candidate points by using information indicating which side of leg to which a motion candidate point belongs (hereinafter, refer to as leg side).

The time-feature extracting unit 14 extracts a time feature of the two motion candidate points (step S32). That is, the time-feature extracting unit 14 extracts a time interval of successive the two motion candidate points by using time information.

The phase-feature extracting unit 15 extracts a phase feature of the two motion candidate points (step S33). That is, the phase-feature extracting unit 15 extracts a phase feature indicating whether rotations in the traverse direction are the same direction, for the successive two motion candidate points.

The direction-change-section determining unit 16 determines whether the index i is smaller than a total number M of motion candidates (step S34). When the index i is smaller than the total number M of motion candidate points (step S34: YES), the direction-change-section determining unit 16 adds 1 to the index i (step S35), and shifts to step S30 to process the next index i.

On the other hand, when the index i is equal to or larger than the total number M of motion candidate points (step S34: NO), the direction-change-section determining unit 16 acquires determination parameters (step S36).

The direction-change-section determining unit 16 determines a section in which motion candidate points of the left leg and the right leg occur "alternately", "successively", and "in the same phase" as a direction change section, by using the determination parameters (step S37). The direction-change-section determining unit 16 ends the direction-change-section extraction processing.

[Flowchart of Direction-Change-Feature Extraction Processing]

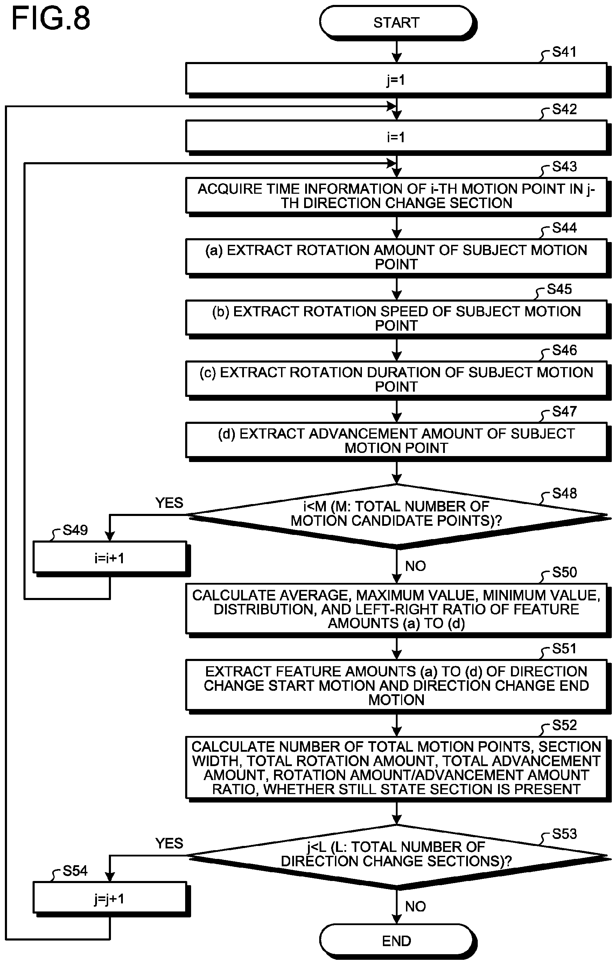

Next, a flowchart of direction-change-feature extraction processing according to the embodiment is explained, referring to FIG. 8. FIG. 8 is a flowchart of direction-change feature-amount-extraction processing according to the embodiment.

As illustrated in FIG. 8, the direction-change-feature extracting unit 17 sets the initial value 1 to an index j (step S41). j is the index indicating a direction change section. The direction-change-feature extracting unit 17 sets the initial value 1 to the index i (step S42). i is the index indicating a motion candidate point.

The direction-change-feature extracting unit 17 acquires time information of a i-th subject motion point in a j-th direction change section from various kinds of data stored in the data storage unit 23 (step S43). (a) A rotation amount of at the subject motion point is extracted (step S44). For example, the rotation amount of the subject motion point can be acquired by calculating an integrated value of gyro in the traverse direction from a start time until an end time at the subject motion point, as one example. The direction-change-feature extracting unit 17 (b) extracts a rotational speed at the subject motion point (step S45). For example, the rotational speed of the subject motion point can be acquired by extracting a maximum value of gyro in the traverse direction from the start time until the end time at the subject motion point. The direction-change-feature extracting unit 17 (c) extracts a rotation duration of the subject motion point (step S46). For example, the rotation duration of the subject motion point can be acquired by calculating a section width from the start time until the end time of the subject motion point. The direction-change-feature extracting unit 17 (d) extracts an advancement amount of the subject motion point (step S47). For example, the advancement amount of the subject motion point can be acquired by extracting an integrated value of gyro in the fore-and-aft direction from the start time until the end time of the subject motion point.

The direction-change-feature extracting unit 17 determines whether the index i is smaller than the total number M of motion candidate points (step S48). When the index i is smaller than the total number M of the motion candidate point (step S48: YES), the direction-change-feature extracting unit 17 adds 1 to the index i (step S49), and shifts to step S43 to process the next index i.

On the other hand, when the index i is equal to or larger than the total number M of the motion candidate point (step S48: NO), the direction-change-feature extracting unit 17 calculates a statistic of the direction change section j as follows. The direction-change-feature extracting unit 17 calculates an average value, a maximum value, a minimum value, a distribution, and a left-right ratio of the respective feature amounts (a) to (d) (step S50). The direction-change-feature extracting unit 17 extracts the feature amounts (a) to (d) of a direction-change start motion and a direction-change end motion (step S51). The direction-change start motion is a motion at the first motion candidate point. The direction-change end motion is a motion at the M-th motion candidate point.

In addition, the direction-change-feature extracting unit 17 calculates a total number of motion points, a duration, a total rotation amount, a total advancement amount, a rotation amount/advancement amount ratio, and whether a still state section is present (step S52) .

The direction-change-feature extracting unit 17 determines whether the index j is smaller than a total number L of the direction change section (step S53). When the index j is smaller than the total number L of the direction change section (step S53: YES), the direction-change-feature extracting unit 17 adds 1 to the index j (step S54), and shifts to step S42 to process the next index j.

On the other hand, when the index j is equal to or larger than the total number L of the direction change section (step S53: NO), the direction-change-feature extracting unit 17 ends the direction-change-feature extraction processing.

[Flowchart of Transition-Gait-Section Extraction Processing]

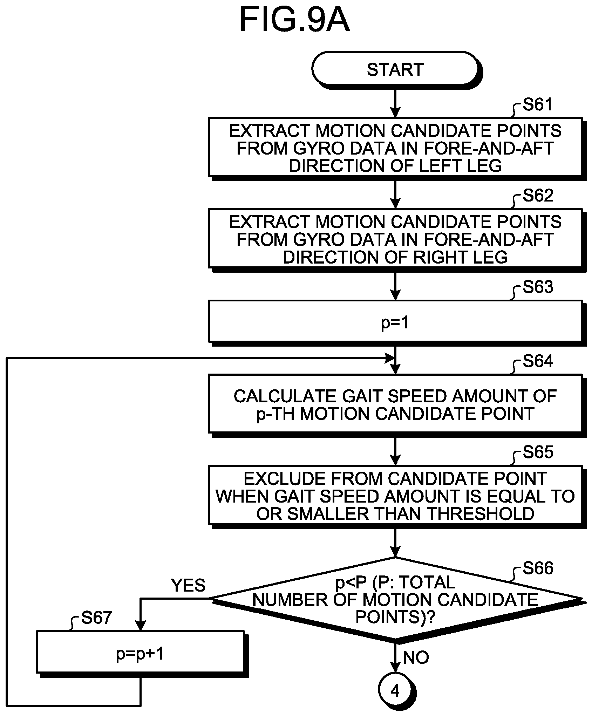

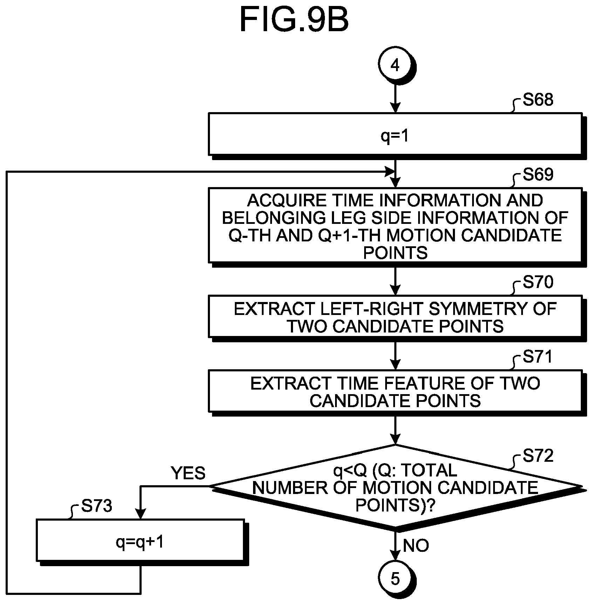

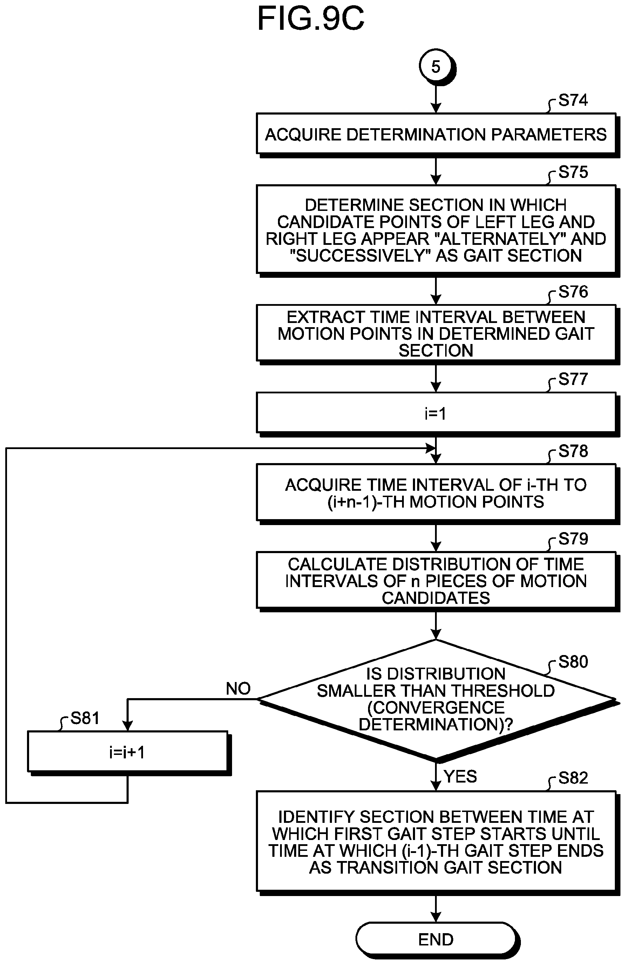

Next, a flowchart of transition-gait-section extraction processing according to the embodiment is explained, referring to FIG. 9A to FIG. 9C. FIG. 9A to FIG. 9C are flowcharts of the transition-gait-section extraction processing according to the embodiment.

As illustrated in FIG. 9A, the gait-section determining unit 19 extracts a motion candidate point from fore-and-aft direction gyro data of the left leg (step S61). For example, the gait-section determining unit 19 detects a peak from the rotation amount in the fore-and-aft direction of the left leg out of data stored in the data storage unit 23.

The gait-section determining unit 19 extracts a motion candidate point from fore-and-aft direction gyro data of the right leg (step S62). For example, the gait-section determining unit 19 detects a peak from the rotation amount in the forward direction of the right leg out of data stored in the data storage unit 23.

The gait-section determining unit 19 sets the initial value 1 to an index p (step S63). The gait-section determining unit 19 calculates a rotation amount (gait speed amount) in the fore-and-aft direction of a p-th motion candidate point (step S64). The gait-section determining unit 19 excludes from the motion candidate point if the gait speed amount is equal to or smaller than a threshold (step S65). The threshold herein is, for example, the threshold R2 described above.

The gait-section determining unit 19 determines whether an index p is smaller than a total number P of motion candidate points (step S66). When the index p is smaller than the total number P of the motion candidate point (step S66: YES), the gait-section determining unit 19 adds 1 to the index p (step S67), and shifts to step S64 to process the next index p.

On the other hand, when the index p is equal to or larger than the total number P of the motion candidate point (step S66: NO), the gait-section determining unit 19 sets the initial value 1 to an index q (step S68). The gait-section determining unit 19 acquires time information and information of a belonging leg at q-th and q+1-th motion candidate points (step S69).

The gait-section determining unit 19 extracts left-right symmetry of two motion candidate points (step S70). That is, the gait-section determining unit 19 extracts a feature indicating whether the left leg and the right leg alternately appear, for successive two motion candidate points by using information of a belonging leg to which a motion candidate point belongs.

The gait-section determining unit 19 extracts a time feature of the two motion candidate points (step S71). That is, the gait-section determining unit 19 extracts a time interval of the successive two motion candidate points by using time information.

The gait-section determining unit 19 determines whether the index q is smaller than a total number Q of motion candidates (step S72). When the index q is smaller than the total number Q of motion candidate points (step S72: YES), the gait-section determining unit 19 adds 1 to the index q (step S73), and shifts to step S69 to process the next index q.

On the other hand, when the index q is equal to or larger than the total number Q of motion candidate points (step S72: NO), the gait-section determining unit 19 acquires determination parameters (step S74).

The gait-section determining unit 19 determines a section in which motion candidate points of the left leg and the right leg occur "alternately" and "successively" as a gait section, by using the determination parameters (step S75). The gait-section determining unit 19 regards motion candidate points in the determined gait section as motion points. The gait-section determining unit 19 then extracts a time interval between motion points in the determined gait section (step S76).

Subsequently, the gait-section determining unit 19 sets the initial value 1 to the index i step (S77). The gait-section determining unit 19 acquires a time interval between adjacent motion points among the i-th to the (i+n-1)-th motion points (step S78). n herein is the number of motion points that are used to determine whether it is a transition gait. n is, for example, 4, but not limited thereto.

The gait-section determining unit 19 calculates a distribution of time intervals of n pieces of motion points (step S79). The gait-section determining unit 19 determines whether the distribution is smaller than a threshold (step S80). When the distribution is equal to or larger than the threshold (step S80: NO), the gait-section determining unit 19 adds 1 to the index i (step S81), and calculates a next distribution. This is because the distribution is not converged.

On the other hand, when the distribution is smaller than the threshold (step S80: YES), the gait-section determining unit 19 identifies a section from a time when the first gait step starts until a time when the (i-1)-th gait step is finished, as a transition gait section (step S82). This is because the distribution has been converged. The gait-section determining unit 19 ends gait-section-identification processing.

[Flowchart of Transition-Gait-Feature Extraction Processing]

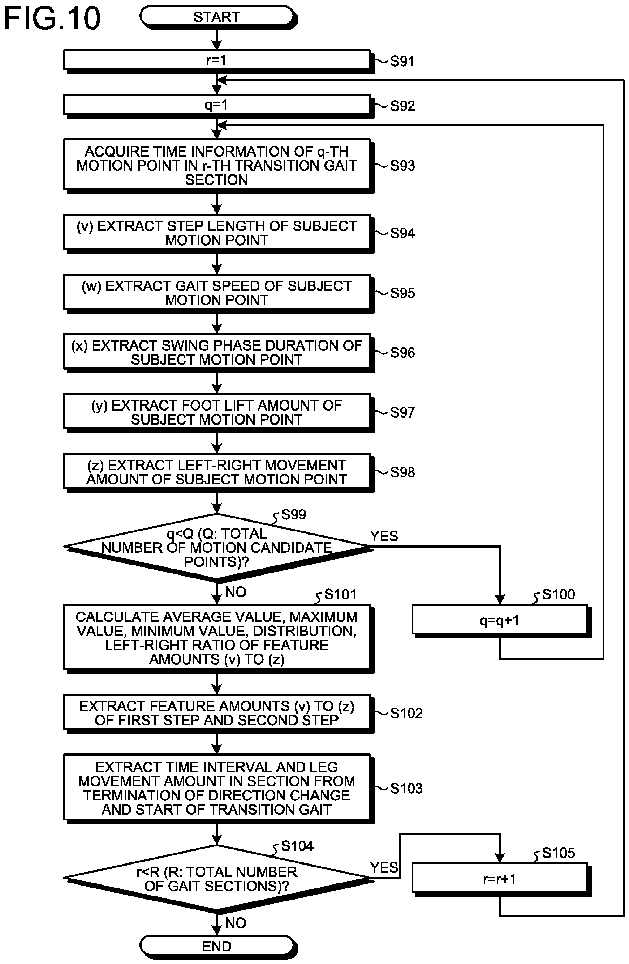

Next, a flowchart of transition-gait-feature extraction processing according to the embodiment is explained, referring to FIG. 10. FIG. 10 is a flowchart of transition-gait-feature extraction processing according to the embodiment.
As illustrated in FIG. 10, the gait-feature extracting unit 20 sets the initial value 1 to index r (step S91). r is the index indicating a gait section. The gait-feature extracting unit 20 sets the initial value 1 to index q (step S92). q is the index indicating a motion point.
The gait-feature extracting unit 20 acquires time information of a q-th subject motion point in an r-th transition gait section from various kinds of data stored in the data storage unit 23 (step S93). The gait-feature extracting unit 20 (v) extracts a step length of a subject motion point (step S94). For example, the step length of the subject motion point can be acquired by calculating an integrated value of gyro in the fore-and-aft direction from a start time until an end time of the subject motion point, and by multiplying the integrated value by a predetermined coefficient. The gait-feature extracting unit 20 (w) extracts a gait speed at the subject motion point (step S95). For example, the gait speed of the subject motion point can be acquired by extracting a maximum value of gyro in the fore-and-aft direction from the start time until the end time of the subject motion point. The gait-feature extracting unit 20 (x) extracts a duration of a swing phase of the subject motion point (step S96). For example, the duration of the swing phase of the subject motion point can be acquired by calculating a section width from the start time until the end time of the subject motion point. The gait-feature extracting unit 20 (y) extracts a foot lift amount of the subject motion point (step S97). For example, the foot lift amount of the subject motion point can be acquired by calculating an integrated value of gyro in the vertical direction from the start time until the end time of the subject motion point. The gait-feature extracting unit 20 (z) extracts a left-right movement amount of the subject motion point (step S98). For example, the left-right movement amount of the subject motion point can be acquired by calculating an integrated value of gyro in a left-and-right direction from the start time until the end time of the subject motion point.
The gait-feature extracting unit 20 determines whether the index q is smaller than the total number Q of motion candidate points (step S99). When the index q is smaller than the total number Q of motion candidate points (step S99: YES), the gait-feature extracting unit 20 adds 1 to the index q (step S100), and shifts to step S93 to process the next index q.
On the other hand, when the index q is equal to or larger than the total number Q of motion candidate points (step S99: NO), the gait-feature extracting unit 20 calculates a statistic of the gait section r as follows. The gait-feature extracting unit 20 calculates an average value, a maximum value, a minimum value, a distribution, and a left-right ratio of the respective feature amounts (v) to (z) (step S101). The gait-feature extracting unit 20 extracts the feature amounts (v) to (z) of the first step and the second step (step S102).
In addition, the gait-feature extracting unit 20 extracts a time interval in a section between an end of the direction change and a start of the transition gait, and a foot movement amount of each leg (step S103).
The gait-feature extracting unit 20 determines whether the index r is smaller than the total number R of the gai sections (step S104). When the index r is smaller than the total number R of the gait sections (step S104: YES), the gait-feature extracting unit 20 adds 1 to the index r (step S105), and shifts to step S92 to process the next index r.
On the other hand, when the index r is equal to or larger than the total number R of the gait sections (step S104: NO), the gait-feature extracting unit 20 ends the gait-feature extraction processing.

[Specific Example of Motion-Candidate Extraction Processing]

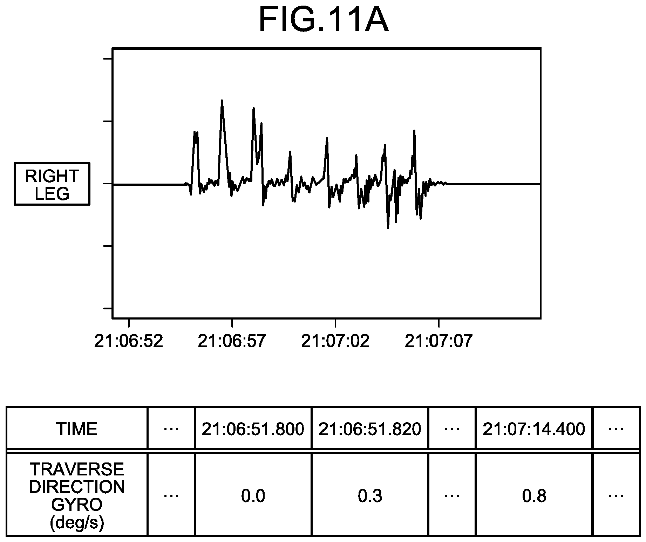

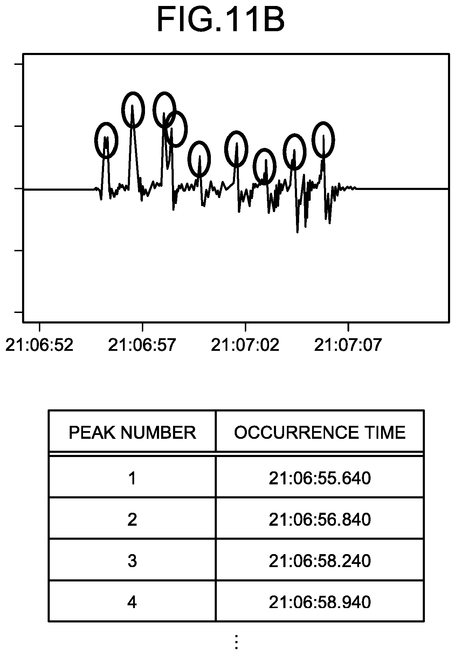

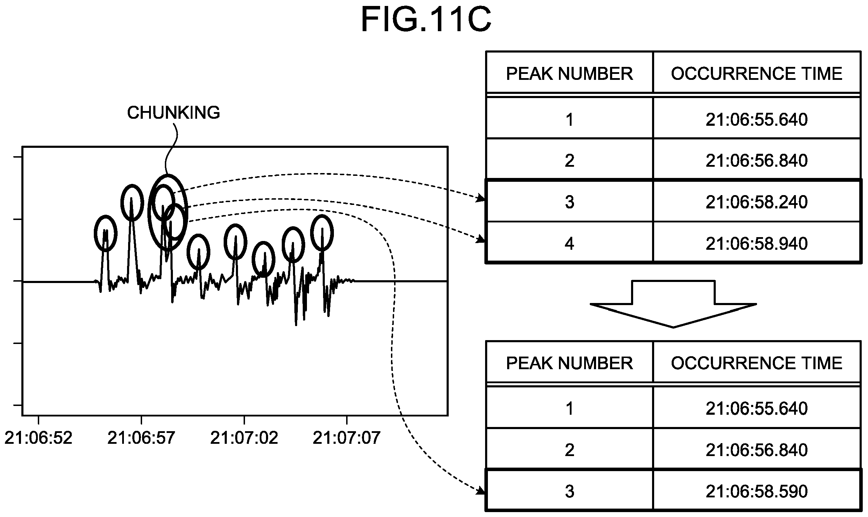

Next, a specific example of motion-candidate extraction processing according to the embodiment is explained, referring to FIG. 11A to FIG. 11E. FIG. 11A to FIG. 11E illustrate a specific example of the motion-candidate extraction processing according to the embodiment.
As illustrated in FIG. 11A, the motion-candidate extracting unit 12 extracts a gyro in the traverse direction of the right leg (angular velocity in the traverse direction) per time from the data storage unit 23. In this example, a value of gyro in the traverse direction at "21:06:51.800" is "0.0 (deg/s). A value of gyro in the traverse direction at "21:06:51.820" is "0.3" (deg/s). A value of gyro in the traverse direction at "21:07:14.400" is "0.8" (deg/s).
As illustrated in FIG. 11B, the motion-candidate extracting unit 12 detects a peak from a rotation amount in the traverse direction. In this example, a peak of a peak number "1" is detected at "21:06:55.640" as the occurrence time. A peak of a peak number "2" is detected at "21:06:56.840" as the occurrence time.
As illustrated in FIG. 11C, the motion-candidate extracting unit 12 performs chunking processing when multiplex peaks are detected. That is, the motion-candidate extracting unit 12 groups, when peaks of rotation in the same traverse direction successively appear in the same leg within predetermined time, these peaks. In this example, a peak of a peak number "3" and a peak of a peak number "4" appear successively in predetermined time. Therefore, the motion-candidate extracting unit 12 groups the peak of the peak number "3" and the peak of the peak number "4" into one unit of peak of the peak number "3".

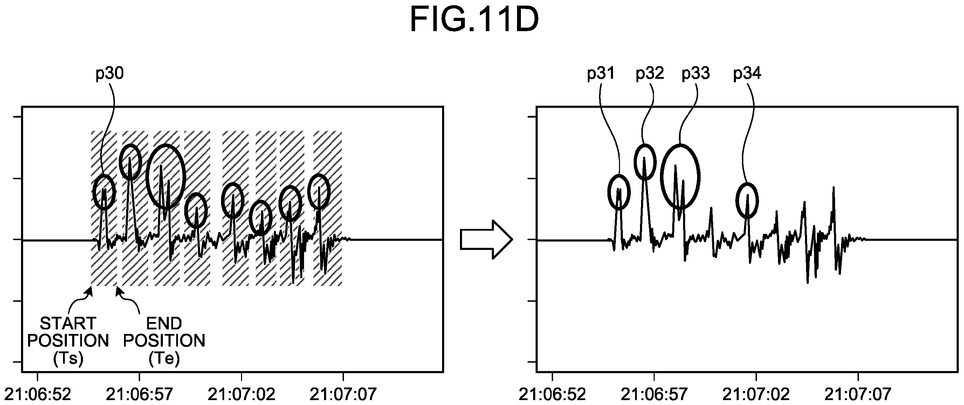

As illustrated in a diagram on the left in FIG. 11D, the motion-candidate extracting unit 12 identifies a start position and an end position of each peak. For example, the motion-candidate extracting unit 12 refers a gyro before the peak and identifies a time at which a gyro change lowers a threshold in a small time width as the start position. The motion-candidate extracting unit 12 refers a gyro after the peak and identifies a time at which a gyro change lowers a threshold in a small time width as the end position. In this example, in a case of a peak indicated by a symbol p30, referring back to a gyro before the peak, a time Ts at which a gyro change lowers a threshold in a small time width is identified as the start position. Referring a gyro after the peak, a time Te at which a gyro change lowers a threshold in a small time width is identified as the end position.

The motion-candidate extracting unit 12 then calculates a gyro integrated value in the traverse direction from the start position to the end position as a rotation amount in the traverse direction for each peak. The rotation amount in the traverse direction is calculated by following Equation (1). i is the peak number. Ts(i) is the peak start position of i-th motion candidate point. Te(i) is the peak end position of the i-th motion candidate point. r(i) is a rotation amount in the traverse direction of the i-th motion candidate point.

$$r^{(i)} = \sum_{t \subset [Ts(i), Te(i)]} gyroY(t) \qquad \text{Eq. (1)}$$

As illustrated in a right diagram in FIG. 11D, the motion-candidate extracting unit 12 determines whether a rotation amount in the traverse direction is larger than the first threshold for each peak. The motion-candidate extracting unit 12 extracts, when the rotation amount in the traverse direction is larger than the first threshold, such a peak as the motion candidate point. In this example, the motion-candidate extracting unit 12 extracts peaks of p31, p32, p33, and p34 as the motion candidate points for the right leg.

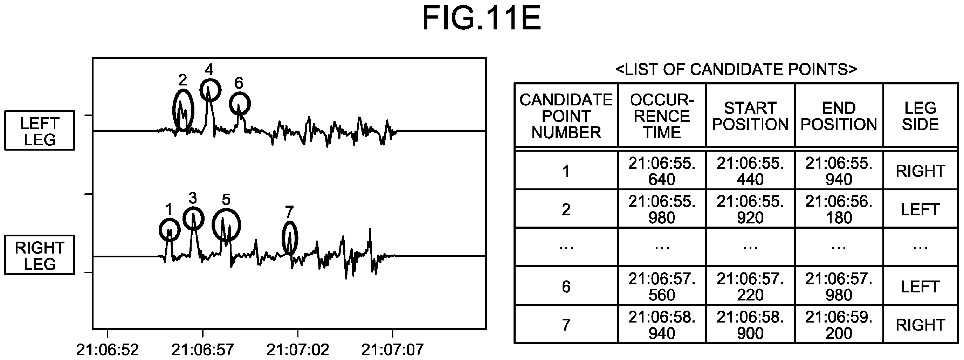

As illustrated in FIG. 11E, the motion-candidate extracting unit 12 performs the same processing indicated in FIG. 11A to FIG. 11D for the left leg, and extracts the motion candidate point. The motion candidate points of the right leg and the motion candidate points of the left leg are expressed in a graph in the left. Numerals above the motion candidate points expressed in the graph are candidate point numbers. The motion candidate points of the right leg and the motion candidate points of the left leg are given in a table in the right. In the table, the occurrence time, the start position, the end position, and the leg side are associated with the candidate point number. As an example, when the candidate point number is "1", "21:06:55.640" is set as the occurrence time, "21:06:55.440" is set as the start position, "21:06:55.940" is set as the end position, and "right" is set as the leg side. When the candidate point number is "2", "21:06:55.980" is set as the occurrence time, "21:06:55.920" is set as the start position, "21:06:56.180" is set as the end position, and "left" is set as the leg side.

[Specific Example of Direction-Change-Section Determination Processing]

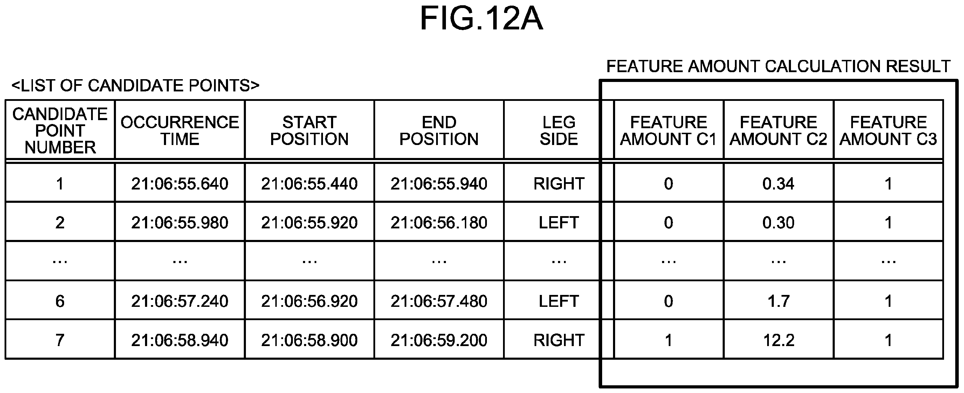

Next, a specific example of direction-change-section determination processing according to the embodiment is explained, referring to FIG. 12A to FIG. 12D. FIG. 12A to FIG. 12D illustrate a specific example of the direction-change-section determination processing according to the embodiment.

As illustrated in FIG. 12A, the left-right-symmetry extracting unit 13 extracts whether the belonging leg is the same as that of the motion candidate point that occurs next as 0/1, for each motion candidate point. AS an example, "0" indicates that the belonging legs are not the same, that is, alternate legs. "1" indicates that the belonging legs are the same. That is, the left-right-symmetry extracting unit 13 extracts a feature amount C1 that expresses that the left leg and the right leg alternately occur of each motion candidate point. The time-feature extracting unit 14 extracts an occurrence time interval until a motion candidate point occurring next, for each motion candidate point. That is, the time-feature extracting unit 14 extracts a feature amount C2 that expresses successive occurrence within predetermine time, for each motion candidate point. The direction-change-section determining unit 16 extracts whether the rotation direction is the same as that of the motion candidate point occurring next as 0/1, for each motion candidate point. "1" indicates that the rotation directions are the same. "0" indicates that the rotation directions are not the same. That is, the direction-change-section determining unit 16 extracts a feature amount C3 that expresses occurrence in the same phase per motion candidate point.

In this example, to the feature amount C3, "0" that indicates it is alternate with respective following motion candidate point numbers is set for the motion candidate point numbers 1 to 6 is set in the table. For the motion candidate number 7, "1" that indicates it is not alternate with the following motion candidate point number is set. To the feature amount C3, "1" that indicates that the rotation direction is the same as that of the respective following motion candidate point numbers, for the motion candidate point numbers 1 to 7 in the table.

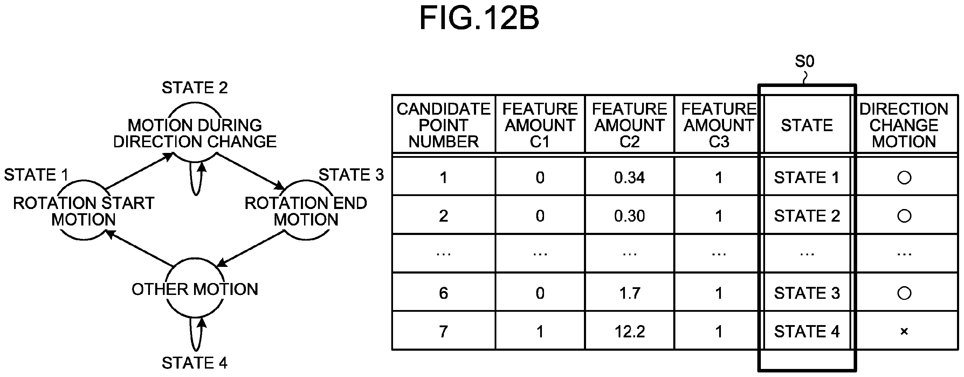

A case in which the direction-change-section determining unit 16 uses the hidden Markov model (HMM) to determine the direction change section is explained. As illustrated in the left of the FIG. 12B, following four states are assumed as hidden states. State 1 is a motion of starting a rotation (direction change). State 2 is a motion during a direction change. State 3 is a motion of finishing a rotation (direction change). State 4 is another motion (non-direction change motion). State 1, state 2, and state 3 correspond to the direction change motion. A state transition model and an observation model are defined in advance. The state transition model in this example is a probability model expressing which state is likely to appear next per state when assuming, in a direction change, a transition of state in motions that a motion of starting a rotation is followed by multiple rotation motions, and finally by a motion of finishing the rotation. The observation model is a probability model expressing what kind of feature amount is observed per state. When the models are defined, it can be determined heuristically or by using learning data. As for the latter case, data when a subject makes a direction change is collected, and model parameters are learned based on the data. For example, in this parameter learning, the Baum-Welch algorithm is applied.

As illustrated in FIG. 12B, the direction-change-section determining unit 16 allocates the most probable state to each motion candidate point, by using the state transition model and the observation model. In this example, an allocated state S0 is set in a table. As state 1, state 2, and state 3 are states of a direction change motion, the direction-change-section determining unit 16 can determine the direction change motion per motion candidate point number by using the allocated state.

A procedure of allocating a state to a candidate point by the direction-change-section determining unit 16 is explained below. The direction-change-section determining unit 16 acquires feature amount $X^{(1)}$, $X^{(2)}$, ..., $X^{(N)}$, and acquires each candidate point state $S=\{S^{(1)}, S^{(2)}, ..., S^{(N)}\}$ by using the HMM. In the HMM used by the direction-change-section determining unit 16, a feature amount of each candidate point is observed only dependent on a state of a candidate point at that time. To estimate a state, a state transition model $p(S^{(i+1)}|S^{(i)})$ and an observation model $p(X^{(i)}|S^{(i)})$ are defined in advance.

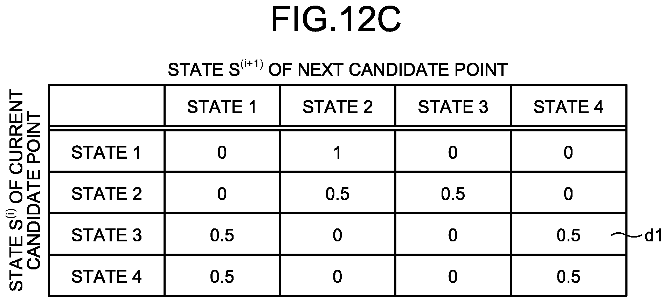

For example, as the state transition model $p(S^{(i+1)}|S^{(i)})$, a transition probability table illustrated in FIG. 12C is defined. The transition probability table expresses a probability per state transition from a state $S^{(i)}$ of the current candidate point to a state $S^{(i+1)}$ of a next candidate point table expressing. As an example, a probability indicated by a symbol d1 that the current candidate point is in the state 3 (motion of finishing a rotation) and the next candidate point is to be in the state 4 (non-direction change) is 0.5. That is, it expresses that $p(S^{(i+1)}=state\ 4|S^{(i)}=state\ 3)$ is 0.5.

Furthermore, the observation model $p(X^{(i)}|S^{(i)})$ is defined by Equation (2). $p(X^{(i)}|S^{(i)})$ expresses a probability of a state S when a feature amount X is observed. In this example, it is assumed that feature amounts are of three kinds of the feature amounts C1, C2, C3 of FIG. 12B, and j=3. $X^{(i)}$ is then expressed by $\{X_1^{(i)}, X_1^{(i)}, X_3^{(i)}\}$. $X_1^{(i)}, X_1^{(i)}, X_3^{(i)}$ are the feature amounts C1, C2, C3 at the i-th candidate point, respectively. $S^{(i)}$ expresses a state at i-th candidate point (as the state number is 4, i is a natural number of 1 to 4).

$$p\left(X^{(i)}\middle|S^{(i)}\right) = \prod_{j=1}^{J} p\left(x_j^{(i)}\middle|S^{(i)}\right)$$

$$\text{Eq. (2)}$$

$$p\left(X^{(i)}\middle|S^{(i)}\right) = N\left(x_1^{(i)}\middle|\mu_1, \sigma_1, S\right) \times N\left(x_2^{(i)}\middle|\mu_2, \sigma_2, S\right) \times N\left(x_3^{(i)}\middle|\mu_3, \sigma_3, S\right)$$

$$\text{Eq. (3)}$$

As the above probability model, a single Gaussian model or a mixture Gaussian model is used. In the following, simply, a case with the single Gaussian model is explained. The first expression of N in Equation (3) expresses the Gaussian model of the feature amount C1 in the state S. The second expression of N expresses the Gaussian model of the feature amount C in the state S. The third expression of N expresses the Gaussian model of the feature amount C3 in the state S.

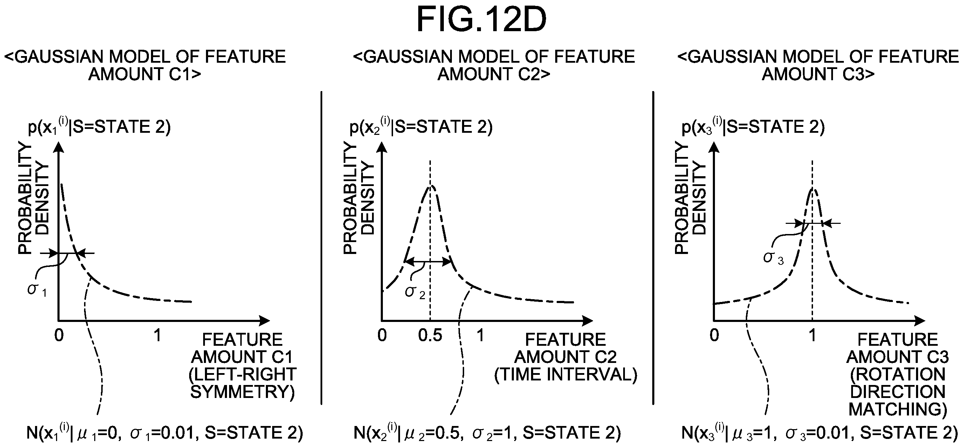

For example, the Gaussian models of the feature amounts C1, C2, and C3 when the state S is a motion during a direction change are illustrated in FIG. 12D. As illustrated in the left in FIG. 12D, in the Gaussian model of the feature amount C1, there is a significantly high possibility that the feature amount C1 is "0" indicating the belonging leg appears alternately on the assumption that the leg on the opposite side moves in the following motion during a direction change. Moreover, as illustrated in the middle in FIG. 12D, in the Gaussian model of the feature amount C2, there is a high possibility that the time interval is small for the feature amount C on the assumption that a time interval until the following motion is small during a direction change. Furthermore, as illustrated in the right in FIG. 12D, in the Gaussian model of the feature amount C3, there is a high possibility that the feature amount C3 is "1" indicating that the rotation directions are the same on the assumption that the rotation direction is the same as that of the following motion during a direction change. That is, it is expressed that there is a high possibility that a next motion is made by an opposite leg, an interval until a next motion is short, and a rotation direction is same as that of a next motion in a motion during a direction change, with Gaussian models.

The direction-change-section determining unit 16 applies the observed feature amounts $X^{(1)}$, $X^{(2)}$, ..., $X^{(N)}$ into the state transition model and the observation model, and estimates the most probable state sequence $S=\{S^{(1)}, S^{(2)}, ..., S^{(N)}\}$. Application of the Viterbi algorithm enables the direction-change-section determining unit 16 to perform this estimation efficiently.

[Specific Example of Direction-Change-Feature Extraction Processing]

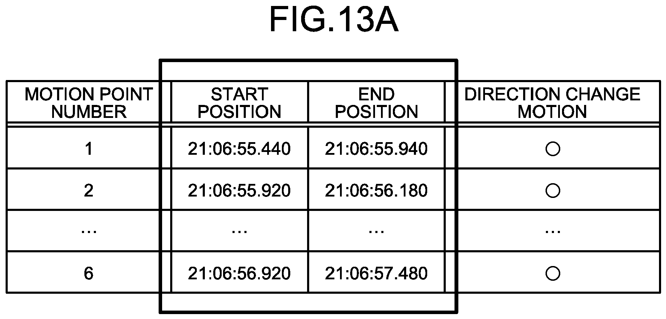

Next, a specific example of direction-change-feature extraction processing according to the embodiment is explained, referring to FIG. 13A to FIG. 13C. FIG. 13A to FIG. 13C illustrate a specific example of the direction-change-feature extraction processing according to the embodiment.

As illustrated in FIG. 13A, the direction-change-feature extracting unit 17 identifies a section of a direction change motion that has been determined by the direction-change-section determining unit 16. That is, the direction-change-feature extracting unit 17 acquires time information of a motion point in the section of the direction change motion.

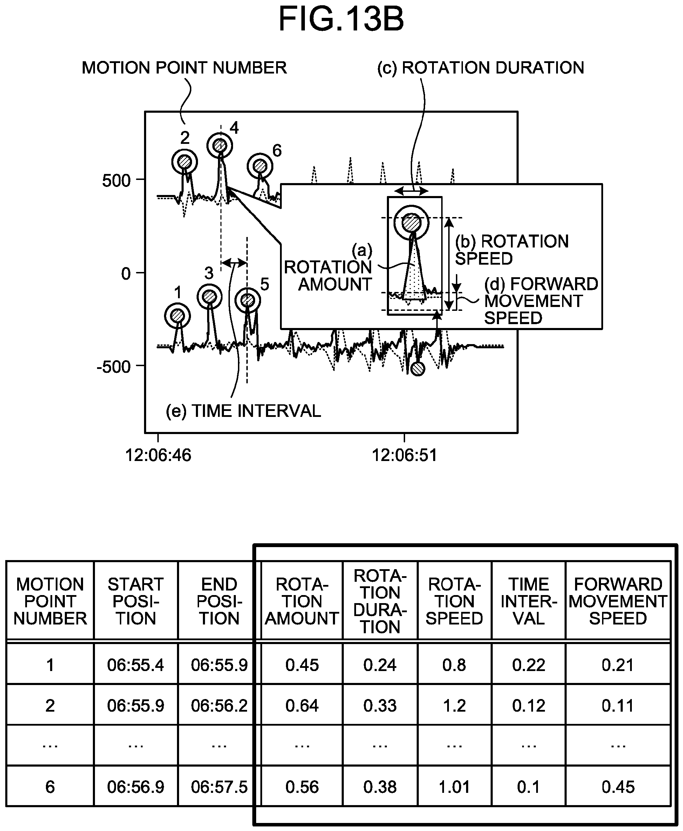

As illustrated in FIG. 13B, the direction-change-feature extracting unit 17 extracts (a) a rotation amount, (b) a rotation speed, (c) a rotation duration, (d) a forward movement speed, and (e) a time interval per motion point in the section of the direction change motion.

For example, as in an upper diagram of FIG. 13B, feature extraction of a motion point of a motion point number "4" is explained. (a) The rotation amount can be acquired by calculating an integrated value of gyro in the traverse direction from a start time until an end time of a motion point. (b) The rotation speed can be acquired by extracting the maximum value of gyro in the traverse direction from a start point until an end point of a motion point. (c) The rotation duration can be acquired by calculating a section width from a start time until an end time of a motion point. (d) The forward movement speed can be acquired by calculating the maximum value of gyro in the fore-and-aft direction from a start time until an end time of a motion point. (e) The time interval can be acquired by calculating a time interval after a rotating motion of a current motion point ends until a next rotating motion starts.

A lower diagram of FIG. 13B indicates extracted results of (a) the rotation amount, (b) the rotation speed, (c) the rotation duration, (d) the forward movement speed, and (e) the time interval for motion point numbers "1" to "6".

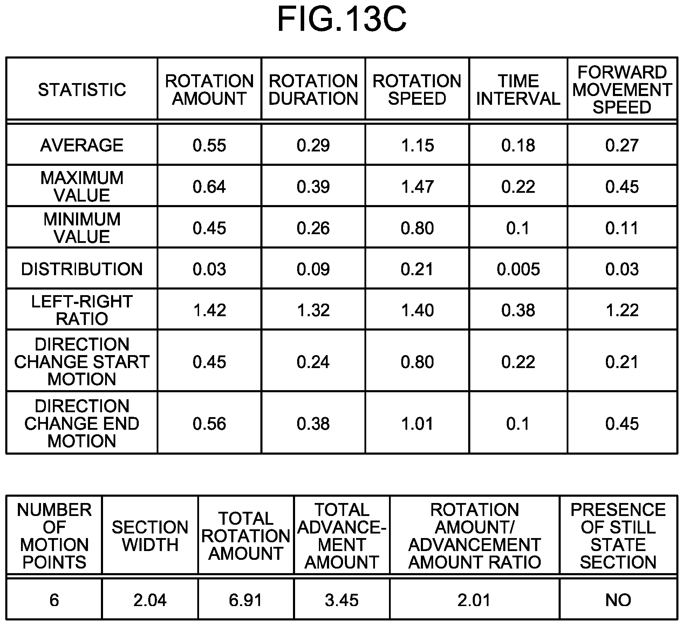

As illustrated in FIG. 13C, the direction-change-feature extracting unit 17 calculates a statistic by using the feature amount of each motion point.

For example, as in the upper diagram of FIG. 13, the direction-change-feature extracting unit 17 calculates an average, a maximum value, a minimum value, a distribution, and a left-right ratio of the respective feature amounts (a) to (e). The direction-change-feature extracting unit 17 extracts the feature amounts (a) to (e) of a direction change start motion and a direction change end motion. The direction change start motion is a motion of a motion point of the motion point number "1". The direction change end motion is a motion of a motion point of the motion point number "6".

In addition, as given in a lower diagram of FIG. 13C, the direction-change-feature extracting unit 17 calculates the number of motion points, a section width, a total rotation amount, a total advancement amount, a rotation amount/advancement amount ratio, and whether a still state section is present.

[Specific Example of Gait-Section Identification Processing]

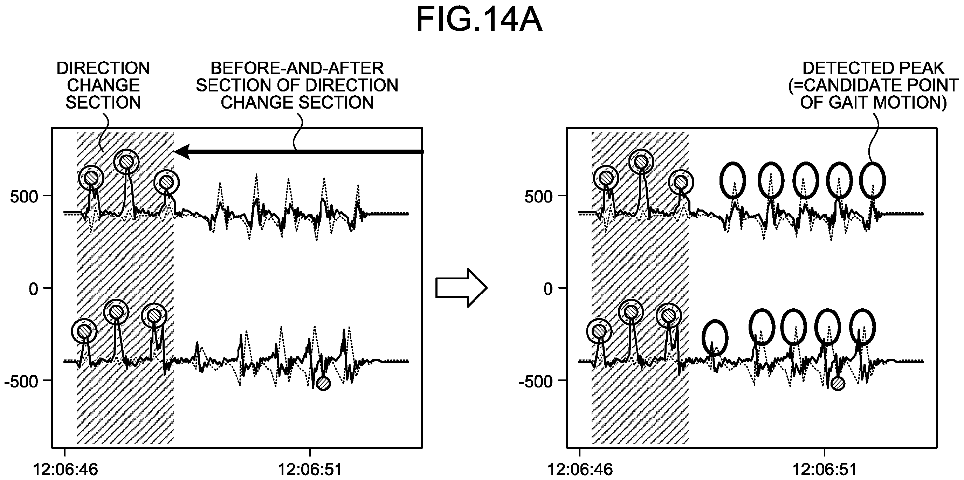

Next, a specific example of gait-section identification processing according to the embodiment is explained, referring to FIG. 14A to FIG. 14C. FIG. 14A to FIG. 14C illustrate a specific example of the gait section identification processing according to the embodiment.

As in an illustration on the left in FIG. 14A, the gait-section determining unit 19 identifies section before and after a direction change section. Subsequently, in an illustration on the right in FIG. 14A, the gait-section determining unit 19 acquires gyro data in the forward direction of both legs in the identified sections and detects peaks. The detected peaks are candidate points of gait motion.

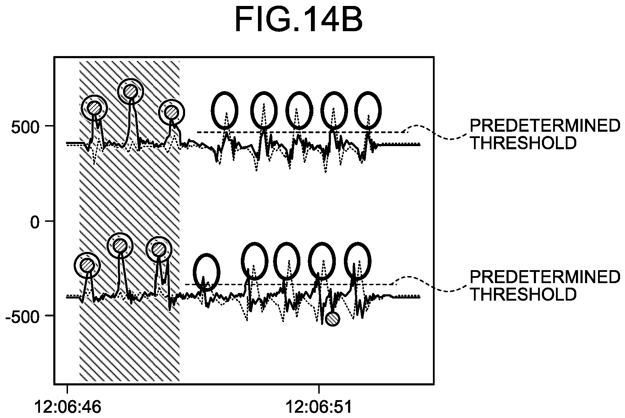

As illustrated in FIG. 14B, the gait-section determining unit 19 determines whether each candidate point includes a swing motion. The gait-section determining unit 19 determines that a swing motion is included when a maximum gait speed (height of the peak) of a candidate point exceeds a predetermined threshold (for example, the threshold R2), and determines as a candidate point. In this example, all the candidate points exceed the predetermined threshold and, therefore, regarded as a candidate point.

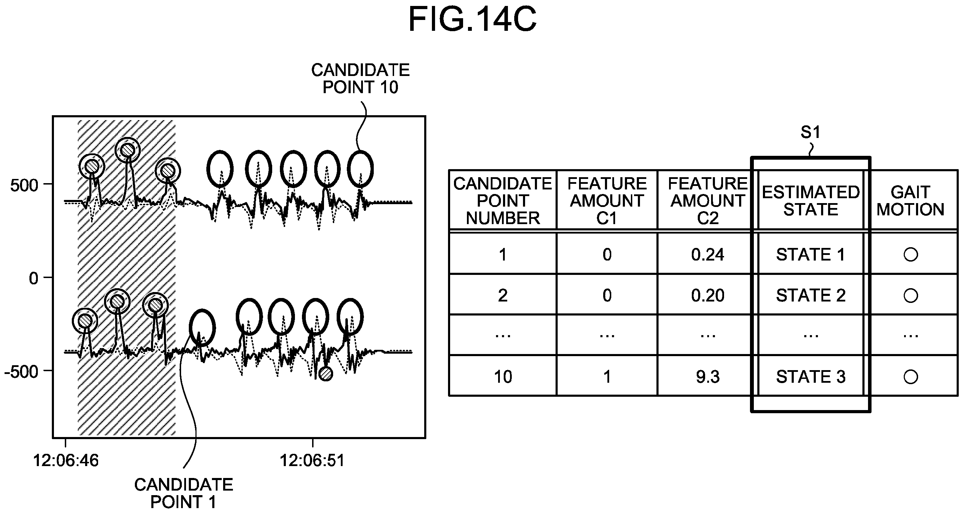

As illustrated in FIG. 14C, the gait-section determining unit 19 identifies a section in which respective candidate points appear "alternately in left and right" and "successively" as a gait section. First, the gait-section determining unit 19 extracts the feature amounts C1 and C2 of each candidate point. That is, the feature amount C1 is extracted as a feature amount indicating that candidate points appear "alternately in left and right". The feature amount C1 is the feature amount expressing that the left leg and right leg alternately appear. The feature amount C2 is extracted as a feature amount indicating that candidate points appear "successively". The feature amount C2 is the feature amount expressing successive appearance in predetermined time.

A case in which the gait-section determining unit 19 uses the hidden Markov model (HMM) to identify a gait section is explained. following four states are assumed as hidden states. State 1 is a motion of starting a gait. State 2 is a motion during a gait. State 3 is a motion of finishing a gait. State 4 is another motion (non-direction change motion). State 1, state 2, and state 3 correspond to the gait motion. A state transition model and an observation model are defined heuristically or by using learning data. The gait-section determining unit 19 allocates the most probable state to each candidate point by using the state transition model and the observation model. In this example, an allocated state S1 is set in a table. As state 1, state 2, and state 3 are states of a gait motion, the gait-section determining unit 19 can determine the gait motion per candidate point number by using the allocated state. As the detailed application process is the same as that in the case of determining the direction change motion, explanation thereof is omitted.

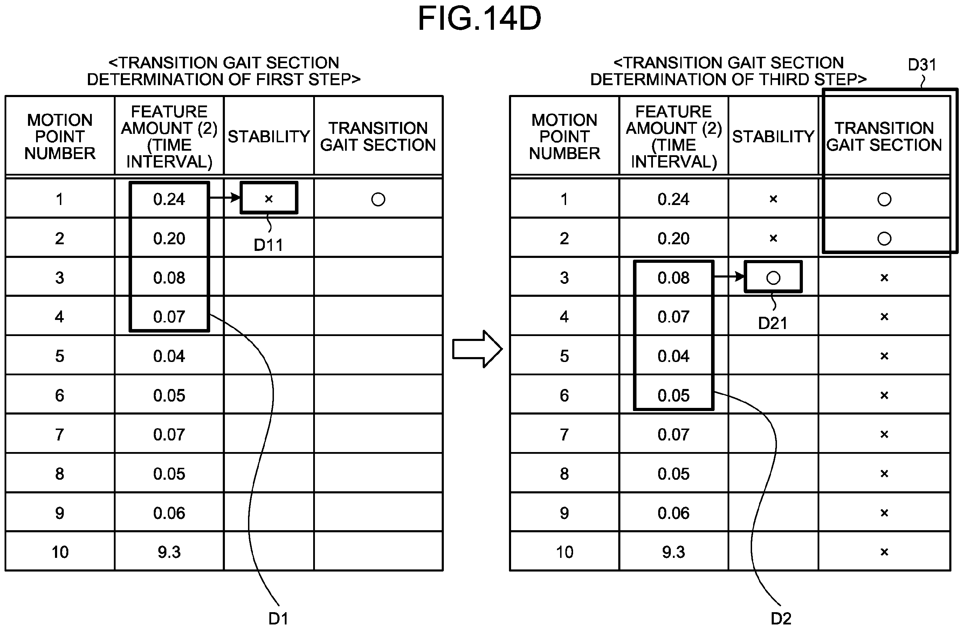

As illustrated in FIG. 14D, the gait-section determining unit 19 identifies a transition section. The transition gait section is a section of unstable walking until the walking is stabilized. A gait feature amount includes, for example, a time interval and a gait speed. In this example, a case in which a time interval is used as the gait feature amount is explained. The gait-section determining unit 19 calculates time intervals of four steps from a current motion point, and calculates a distribution of the time intervals of the four steps. The gait-section determining unit 19 determines that the gait has been stabilized when the distribution is smaller than a threshold, and identifies a section from a motion point of the first step in the section identified as the gait section to a motion point before the current motion point as the transition gait section. In this example, assuming that the current motion point is the motion point of the motion point number "1", the distributions of time intervals of four steps from the current motion point vary (D1). Therefore, an entry of stability for the current motion point is "x" indicating that it is not stable (D11). On the other hand, if the current motion point is a motion point of the motion point number "3", the distributions of time intervals of four steps from the current motion point do not vary (D2). Therefore, the entry of stability of the current point is "o" indicating that it is stable (D21). Thus, the gait-section determining unit 19 identifies a section from the motion point number "1" to the motion point number "2" as the transition gait section (D31).

[Specific Example of Gait-Feature Extraction Processing]

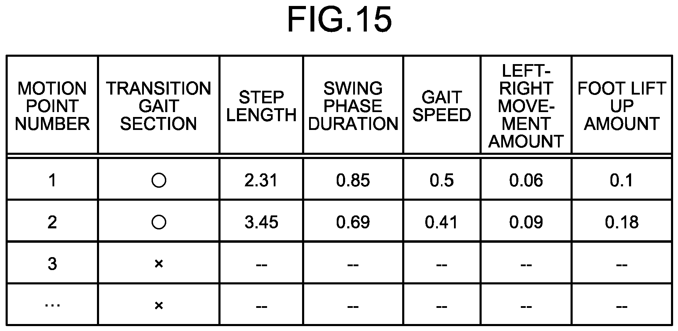

Next, a specific example of gait-feature extraction processing according to the embodiment is explained, referring to FIG. 15. FIG. 15 illustrates a specific example of the gait-feature extraction processing according to the embodiment.

As illustrated in FIG. 15, the gait-feature extracting unit 20 extracts a feature amount of a motion point in the transition gait section that has been identified by the gait-section determining unit 19. The gait-feature extracting unit 20 extracts (v) a step length, (w) a gait speed, (x) a duration of a swing phase, (y) a foot lift amount, and (z) a left-right movement amount of each motion point in the transition gait section. The (v) step length can be acquired by calculating an integrated value of gyro in the forward direction from a start time until an end time of a motion point. The (w) gait speed can be acquired by extracting a maximum value of gyro in the forward direction from a start time until an end time of a motion point. The (x) duration of the swing phase can be acquired by calculating a section width from a start time until an end time of a motion point. The (y) foot lift amount can be acquired by calculating an integrated value of gyro in an upper limit direction from a start time until an end time of a motion point. The (z) left-right movement amount can be acquired by calculating an integrated value of gyro in a left-and-right direction from a start time until an end time of a motion point.

FIG. 15 illustrates extracted results of (v) the step length, (w) the gait speed, (x) the duration of a swing phase, (y) the foot lift amount, and (z) the left-right movement amount for motion point numbers "1" and "2". The gait-feature extracting unit 20 can calculate a statistic by using the feature amount of each motion point. For example, the gait-feature extracting unit 20 can calculate an average, a maximum value, a minimum value, a distribution, and a left-right ratio of the respective feature amounts (v) to (z). The gait-feature extracting unit 20 can extract the feature amounts (v) to (z) of the first step and the second step. The gait-feature extracting unit 20 can calculate a section width of the transition gait section and the total number of gait steps (the number of transition gait steps) in the transition gait section.

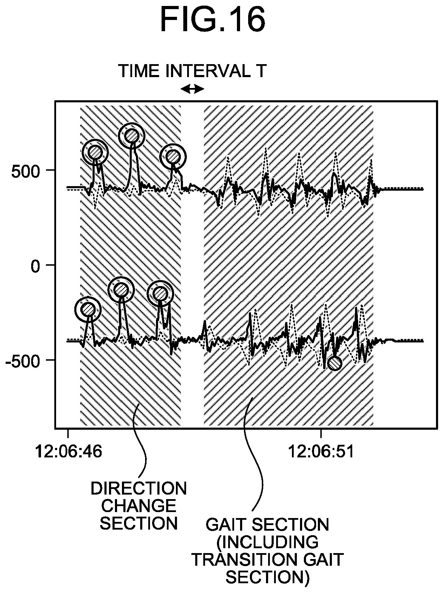

The gait-feature extracting unit 20 can extract a feature amount from between a direction change and a transition gait. FIG. 16 illustrates another specific example of the gait-feature extraction processing according to the embodiment. As illustrated in FIG. 16, the gait-feature extracting unit 20 extracts a feature amount from between a direction change and a transition gait. For example, the gait-feature extracting unit 20 extracts a time interval T from termination of the direction change until start of the transition gait. The gait-feature extracting unit 20 extracts a leg movement amount P of each leg from the termination of the direction change until the start of the transition gait. For the leg movement amount P, a distribution amount of gyro in three axes of each leg in the time interval T is used, as an example.

[Specific Example of Feature-Amount Storage Processing]

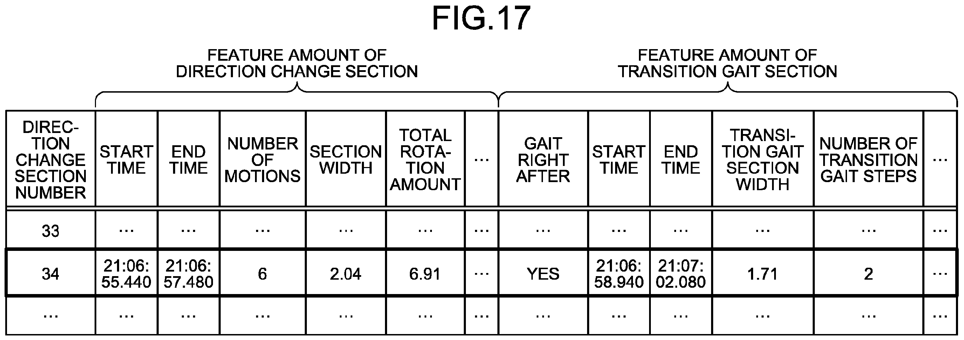

Next, a specific example of feature-amount storage processing according to the embodiment is explained, referring to FIG. 17. FIG. 17 illustrates a specific example of the feature-amount storage processing according to the embodiment.

As illustrated in FIG. 17, the feature-amount storage unit 21 stores a feature amount in a direction change section and a feature amount in a transition gait section in the data storage unit 23 per direction change section. In this example, a result of storing feature amounts in the direction change section and feature amounts in the transition gait section of a direction-change section number "34" is given. The feature amounts in the direction change section are the statistics indicated in the lower diagram in FIG. 13C. The feature amounts in the transition gait section are statistics of the feature amounts of all the motion points illustrated in FIG. 15.

[Specific Example of Feature-Amount Visualization Processing]

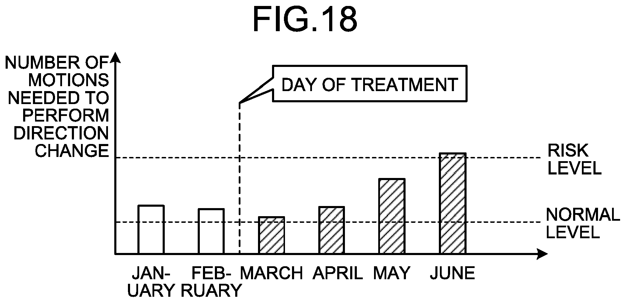

Next, a specific example of feature-amount visualization processing according to the embodiment is explained, referring to FIG. 18. FIG. 18 illustrates a specific example of the feature-amount visualization processing according to the embodiment.

As illustrated in FIG. 18, the feature-amount visualizing unit 22 visualizes various kinds of feature amounts chronologically per month, with reference to a predetermined day, for a section thereafter. In this example, a feature amount to be visualized is the number of motions needed to perform a direction change. To monitor a treatment progress of a patient, the predetermined day is set to a day of treatment. The feature-amount visualizing unit 22 then receives the day of treatment and visualizes the number of motions need to perform a direction change per month, with reference to this day, for sections before and after the section. The number of motions per month to be visualized can be the number of motions on a day that has been determined in each month, or can be an average of the number of motions of each month. Thus, the feature-amount visualizing unit 22 enables to detect that motor deterioration (particularly in a balancing function) is suspected as the number of motions needed to perform a direction change exceeds a risk level in June after the day of treatment, for example.

[Effects of Embodiment]

As described, the information processing apparatus 1 extracts a point at which a motion of leg rotation about an axis of a direction of gravity occurs as a motion candidate in respective data of the sensor device 2 used for a left leg and the sensor device 3 used for a right leg. The information processing apparatus 1 determines extracted motion candidates as a direction change motion when directions of the rotation are the same, a motion candidate of a left leg and a motion candidate of a right leg appear alternately, and appear chronically successively. The information processing apparatus 1 extracts a feature of the direction change motion by using data acquired by the sensors. According to this configuration, the information processing apparatus 1 can extract a feature amount of a direction change motion by using only the sensor devices 2, 3 used for legs on respective sides highly accurately.

Moreover, the information processing apparatus 1 detects a point in which a rotation amount of leg between a time when one foot comes off the ground until a time of landing is larger than a first threshold by using data of at least one axis acquired by the sensor devices 2, 3, and extracts the detected point as a motion candidate of a direction change. According to this configuration, the information processing apparatus 1 can extract a motion candidate of a direction change that is used to extract a feature amount of a direction change motion by using only the sensor devices 2, 3 used for legs on respective sides.

Furthermore, the information processing apparatus 1 groups, when there are points that appear successively in the same leg in predetermined time in a point extracted as a motion candidate, the successive points as one unit, and extracts the grouped point as one motion candidate of a direction change. According to this configuration, the information processing apparatus 1 can extract a motion candidate of one step when a leg on the same side is turned to make a direction change even when the leg on the same side is unstable.

Moreover, the information processing apparatus 1 extracts information including at least one of a rotation amount in the traverse direction, a rotation speed, a rotation duration, and an advancement amount to the forward direction, as a feature amount of a direction change motion, for a point of a direction change motion. According to this configuration the information processing apparatus 1 can extract a feature amount of a direction change motion highly accurately.

Furthermore, the information processing apparatus 1 determines a point in which a rotation amount in the traverse direction is smaller than the first threshold and larger than the second threshold that is smaller than the first threshold as a mild direction change motion, out of points occurring right after a determined direction change motion. The information processing apparatus 1 extracts a feature amount of the determined mild direction change motion by using respective data of the sensor devices 2, 3. According to this configuration, the information processing apparatus 1 determines a mild direction change motion that is estimated to be walking while turning to change a direction, thereby enabled to extract a feature amount of a mild direction change highly accurately.

Moreover, the information processing apparatus 1 extracts a point in which a rotation amount to the forward direction is equal to or larger than the threshold R1 in a section between termination of a direction change motion until start of a next direction change motion as a motion candidate of a gait. The information processing apparatus 1 determines points that are larger than the threshold R2 smaller than the threshold R1 as a start and an end of a gait section, in before and after the extracted motion candidate of a gait. According to this configuration, the information processing apparatus 1 can determine a gait section accurately by determining a start and an end of the gait section.

Furthermore, the information processing apparatus 1 determines sections before a section in which a distribution of time intervals of the predetermined number of sequential points become equal to or lower than a threshold as a transition gait section that indicates that it is an unstable gait section, in a gait section. According to this configuration, the information processing apparatus 1 can determine a transition gait section until it becomes a stable gait in which walking is stable accurately.

Moreover, the information processing apparatus 1 extracts a feature amount of a transition gait section by using data of the respective sensor devices 2, 3. According to this configuration, the information processing apparatus 1 can extract a feature amount of a transition gait section highly accurately.

[Others]

It has been explained that the information processing apparatus 1 extracts a feature amount of a direction change motion and a gait motion of a subject by using data of the sensor devices 2, 3 that are worn on respective legs of the subject. However, the processing of extracting a feature amount of a direction change motion and a gait motion of a subject can be performed by the sensor device 2 or the sensor device 3. For example, the sensor device 2 can be configured to extract a feature amount of a direction change motion and a gait motion of a subject by using data acquired by itself and by the sensor device 3.

Furthermore, in the above embodiment, respective components of the illustrated devices are not necessarily physically configured as illustrated. That is, specific forms of distribution and integration of the respective devices are not limited to the ones illustrated, and all or a part thereof can be configured to be distributed or integrated functionally or physically in arbitrary units according to various kinds of loads, usage conditions, and the like. For example, the gait-section determining unit 19 can be distributed to a determining unit that determines a stable gait section, and a determining unit that determines a transition gait section. The before-and-after-section identifying unit 18 can be distributed to a determining unit that determines a before-and-after section and an extracting unit that extracts a feature amount of the before-and-after section. Furthermore, the left-right-symmetry extracting unit 13, the time-feature extracting unit 14, and the phase-feature extracting unit 15 can be integrated. Moreover, it can be configured to connect the data storage unit 23 to the information processing apparatus 1 as an external device through a network.

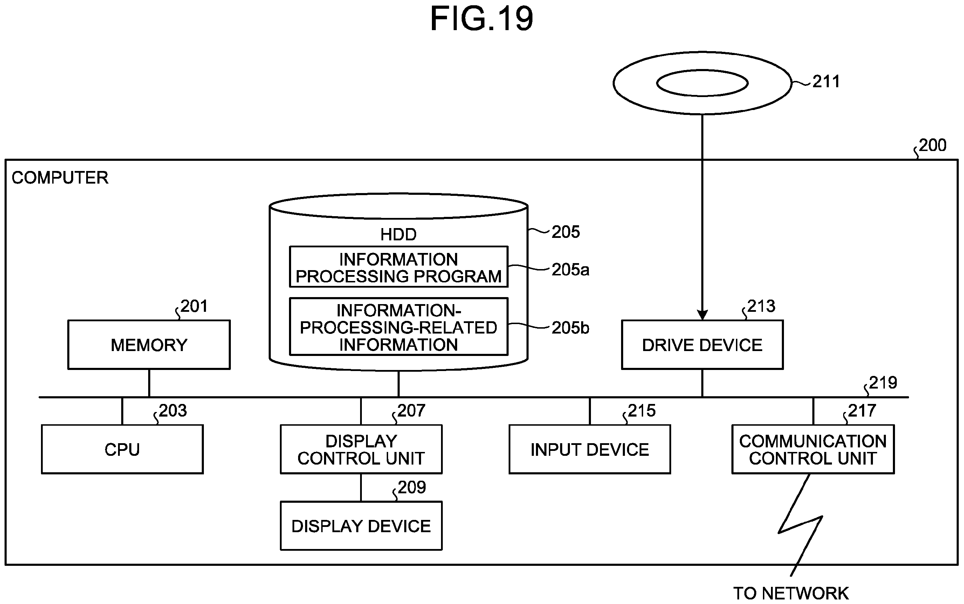

Moreover, the various kinds of processing explained in the above embodiment can be implemented by executing a program that has been prepared in advance by a computer such as a personal computer and a workstation. Therefore, in the following, one example of a computer that executes an information processing program implementing functions similar to those of the information processing apparatus 1 illustrated in FIG. 1 is explained. FIG. 19 illustrates one example of a computer that executes the information processing program.

As illustrated in FIG. 19, a computer 200 includes a CPU 203 that executes various kinds of arithmetic processing, an input device 215 that accepts an input of data from a user, and a display control unit 207 that controls a display device 209. Furthermore, the computer 200 includes a drive device 213 that reads a program and the like from a recording medium, and a communication control unit 217 that communicates data with other computers through a network. Moreover, the computer 200 includes a memory 201 that stores various kinds of information temporarily, and an HDD 205. The memory 201, the CPU 203, the HDD 205, the display control unit 207, the drive device 213, the input device 215, and the communication control unit 217, are connected to each other through a bus 219.

The drive device 213 is, for example, a device for a removable disk 211. The HDD 205 stores an information processing program 205a and information-processing-related information 205b.

The CPU 203 reads the information processing program 205a, and develops it in the memory 201 to execute it as a process. The process corresponds to the respective functional units of the information processing apparatus 1. The information-processing-related information 205b corresponds to the data storage unit 23. For example, the removable disk 211 stores various information such as the information processing program 205a.

The information processing program 205a is not necessarily stored in the HDD 205 from the beginning. For example, it can be stored in a "portable physical medium", such as a flexible disk (FD), a CD-ROM, a DVD, a magnetooptical disk, or an IC card, that is inserted into the computer 200, and the computer 200 can read the information processing program 205a from these to execute it.

[Explanation of Reference]

| | |
|---|---|
| 1 | INFORMATION PROCESSING APPARATUS |
| 2, 3 | SENSOR DEVICE |
| 9 | INFORMATION PROCESSING SYSTEM |
| 11 | DATA ACQUIRING UNIT |
| 12 | MOTION-CANDIDATE EXTRACTING UNIT |
| 13 | LEFT-RIGHT-SYMMETRY EXTRACTING UNIT |
| 14 | TIME-FEATURE EXTRACTING UNIT |
| 15 | PHASE-FEATURE EXTRACTING UNIT |
| 16 | DIRECTION-CHANGE-SECTION DETERMINING UNIT |
| 17 | DIRECTION-CHANGE-FEATURE EXTRACTING UNIT |
| 18 | BEFORE-AND-AFTER-SECTION IDENTIFYING UNIT |
| 19 | GAIT-SECTION DETERMINING UNIT |
| 20 | GAIT-FEATURE EXTRACTING UNIT |
| 21 | FEATURE-AMOUNT STORAGE UNIT |
| 22 | FEATURE-AMOUNT VISUALIZING UNIT |
| 23 | DATA STORAGE UNIT |

**Claims**

**1.** An information processing system (9) comprising:

a first motion sensor (2) that is used for a left leg of a user;
a second motion sensor (3) that is used for a right leg of the user,
wherein the first motion sensor (2) and the second motion sensor (3) each comprises a gyro sensor configured to acquire data of an angular velocity around three axes that are perpendicular to each other; and
an information processing unit (1) configured to process the data acquired by the first motion sensor and the second motion sensor, wherein the information processing unit (1) includes
a motion-candidate extracting unit (12) configured to extract a plurality of peaks in first data among the acquired data from the first motion sensor (2), and in second data among the acquired data from the second motion sensor (3), as motion candidates of a direction change, wherein the peaks indicate a rotation amount of the left leg or the right leg, respectively, in a direction transverse to a walking direction of the user, wherein the rotation amount of the left leg or the right leg indicated by the peaks is larger than a first threshold from a time at which one foot comes off a ground until a time of landing wherein,
when more than one of said peaks successively appear in the first data or the second data in a predetermined time on the same leg, extract the more than one of said peaks as a single motion candidate of one step;
a direction-change-motion determining unit (16) configured to determine a motion indicated by the motion candidates extracted by the motion-candidate extracting unit (12) as a direction change motion based on whether the first data and the second data indicate that a motion candidate of the left leg and a motion candidate of the right leg alternate in temporally appearing therein, and based on whether signs of the rotation amounts of the alternating motion candidates are the same, wherein the direction-change-motion determining unit (16) is configured to determine the motion indicated by the motion candidates extracted by the motion-candidate extracting unit (12) as the direction change motion when the signs of the rotation amounts of the alternating motion candidates are the same, and when the first data and the second data indicate that the motion candidate of the left leg and the motion candidate of the right leg alternate in temporally appearing therein; and
a feature extracting unit (17) configured to extract a feature of the direction change motion that is determined by the direction-change-motion determining unit (16), by using the data acquired by the first motion sensor (2) and the second motion sensor (3).

**2.** The information processing system (9) according to claim 1, wherein the feature extracting unit is configured to extract information that includes at least one of a rotation amount in the left-right direction, a rotation speed, a rotation duration, and an advancement amount to a forward direction as the feature of the direction change motion.

**3.** The information processing system (9) according to claim 1 or claim 2, further including

a mild-direction-change-motion determining unit (16) configured to determine a motion indicated by a plurality of peaks, a rotation amount in the left-right direction of a peak among the peaks being smaller than said first threshold and larger than a second threshold that is smaller than the first threshold, as a mild direction change motion, the plurality of peaks being extracted right after the direction change motion determined by the direction-change-motion determining unit (16), wherein

the feature extracting unit is configured to extract a feature of the mild direction change motion determined by the mild-direction-change-motion determining unit by using the respective data of the first motion sensor (2) and the second motion sensor (3).

4. The information processing system (9) according to any of the preceding claims, further including a gait-section determining unit configured to extract peaks in the first data and the second data, a rotation amount in a forward direction indicated by a peak among the peaks being equal to or larger than a third threshold in a section from termination of the direction change motion until start of a next direction change motion, as motion candidates of a gait, and determine a peak, the rotation amount indicated by the peak being smaller than said third threshold and equal to or larger than a fourth threshold that is smaller than the third threshold, the peak appearing before and after the extracted motion candidates of the gait, as start or end of a gait section indicated by the extracted peaks.

5. The information processing system (9) according to claim 4, wherein the gait-section determining unit is configured to determine, in the gait section, a section in which a distribution of sequential time intervals of a predetermined number is larger than a threshold, as a transition gait section that indicates an unstable gait section.

6. The information processing system (9) according to claim 5, wherein the feature extracting unit is configured to extract a feature of the transition gait section by using the respective data of the first motion sensor (2) and the second motion sensor (3).

7. The information processing system (9) according to any of the preceding claims, wherein the first motion sensor (2) and the second motion sensor (3) each comprise an acceleration sensor.

8. The information processing system (9) according to any of the preceding claims, wherein the information processing unit (1) is implemented by any one of the first motion sensor (2) and the second motion sensor (3).

9. An information processing method in which a computer (200) executes processing of:

   extracting, in a system in which a first motion sensor is used for a left leg of a user, and a second motion sensor is used for a right leg of the user, the first motion sensor and the second motion sensor each comprising a gyro sensor configured to acquire data of an angular velocity around three axes that are perpendicular to each other, a plurality of peaks in first data among acquired data from the first motion sensor, and in second data among the acquired data from the second motion sensor, as motion candidates of a direction change, wherein the peaks indicate a rotation amount of the left leg or the right leg, respectively, in a direction transverse to a walking direction of the user, wherein the rotation amount indicated by the peaks is larger than a first threshold from a time at which one foot comes off a ground until a time of landing,
   wherein, when more than one of said peaks successively appear in the first data or the second data in a predetermined time on the same leg, extracting the more than one of said peaks as a single motion candidate of one step;
   determining a motion indicated by the extracted motion candidates as a direction change motion based on whether the first data and the second data indicate that a motion candidate of the left leg and a motion candidate of the right leg alternate in temporally appearing therein, and based on whether signs of the rotation amounts of the alternating motion candidates are the same, including determining the motion indicated by the extracted motion candidates as the direction change motion when the signs of the rotation amounts of the alternating motion candidates are the same, and when the first data and the second data indicate that the motion candidate of the left leg and the motion candidate of the right leg alternate in temporally appearing therein; and
   extracting a feature of the direction change motion that is determined at the determining, by using the data acquired by the first motion sensor and the second motion sensor.

10. A program which, when executed by a computer, causes the computer to carry out the method according to claim 9.

**Patentansprüche**

1. Informationsverarbeitungssystem (9), umfassend:

   einen ersten Bewegungssensor (2), der für ein linkes Bein eines Benutzers verwendet wird;
   einen zweiten Bewegungssensor (3), der für ein rechtes Bein des Benutzers verwendet wird,
   wobei der erste Bewegungssensor (2) und der zweite Bewegungssensor (3) jeweils einen Gyrosensor umfassen, der dazu ausgelegt ist, Daten einer Winkelgeschwindigkeit um drei senkrecht zueinander stehende Achsen zu erfassen; und
   eine Informationsverarbeitungseinheit (1), die dazu ausgelegt ist, die von dem ersten Bewegungssensor und dem zweiten Bewegungssensor erfassten Daten zu verarbeiten, wobei die Informationsverarbeitungseinheit (1) aufweist
   eine Bewegungskandidaten-Extraktionseinheit (12), die dazu ausgelegt ist, mehrere Peaks in ersten Daten unter den von dem ersten Bewegungssensor (2) erfassten Daten und in zweiten Daten unter den von dem zweiten Bewegungssensor (3) erfassten Daten erfassten Daten als Bewegungskandidaten einer Richtungsänderung zu extrahieren, wobei die Peaks einen Rotationsbetrag des linken Beins bzw. des rechten Beins in einer Richtung quer zu einer Laufrichtung des Benutzers anzeigen, wobei der durch die Peaks angezeigte Rotationsbetrag des linken Beins oder des rechten Beins von einem Zeitpunkt an, zu dem ein Fuß vom Boden abhebt, bis zu einem Zeitpunkt der Landung größer als eine erster Schwellenwert ist, wobei, wenn mehr als einer der Peaks in den ersten Daten oder den zweiten Daten nacheinander erscheint, bei demselben Bein, in einer vorbestimmten Zeit, der mehr als eine der Peaks als ein einzelner Bewegungskandidat eines Schritts extrahiert wird;
   eine Richtungsänderungsbewegungs-Bestimmungseinheit (16), die dazu ausgelegt ist, eine Bewegung, die von den durch die Bewegungskandidaten-Extraktionseinheit (12) extrahierten Bewegungskandidaten angezeigt wird, als Richtungsänderungsbewegung zu bestimmen, basierend darauf, ob die ersten Daten und die zweiten Daten anzeigen, dass sich ein Bewegungskandidat des linken Beins und ein Bewegungskandidat des rechten Beins abwechseln, zeitlich darin zu erscheinen, und basierend darauf, ob die Vorzeichen der Rotationsbeträge der abwechselnden Bewegungskandidaten gleich sind, wobei die Richtungsänderungsbewegungs-Bestimmungseinheit (16) dazu ausgelegt ist, die Bewegung, die von den durch die Bewegungskandidaten-Extraktionseinheit (12) extrahierten Bewegungskandidaten angezeigt wird, als Richtungsänderungsbewegung zu bestimmen, wenn die Vorzeichen der Rotationbeträge der abwechselnden Bewegungskandidaten gleich sind, und wenn die ersten Daten und die zweiten Daten anzeigen, dass der Bewegungskandidat des linken Beins und der Bewegungskandidat des rechten Beins sich abwechseln, zeitlich darin zu erscheinen; und
   eine Merkmalsextraktionseinheit (17), die dazu ausgelegt ist, unter Verwendung der von dem ersten Bewegungssensor (2) und der zweiten Bewegungssensor (3) erfassten Daten ein Merkmal der durch die Richtungsänderungsbewegungs-Bestimmungseinheit (16) bestimmten Richtungsänderungsbewegung zu extrahieren.

2. Informationsverarbeitungssystem (9) nach Anspruch 1, wobei die Merkmalsextraktionseinheit dazu ausgelegt ist, Informationen zu extrahieren, die mindestens einen Rotationsbetrag in der Links-Rechts-Richtung, eine Rotationsgeschwindigkeit, eine Rotationsdauer, und einen Fortschrittsbetrag in einer Vorwärtsrichtung als das Merkmal der Richtungsänderungsbewegung aufweisen.

3. Informationsverarbeitungssystem (9) nach Anspruch 1 oder Anspruch 2, ferner aufweisend eine Bestimmungseinheit (16) für eine leichte Richtungsänderungsbewegung, die dazu ausgelegt ist, eine Bewegung, die durch mehrere Peaks angezeigt wird, wobei ein Rotationsbetrag in der Links-Rechts-Richtung eines Peaks unter den Peaks kleiner ist als der erste Schwellenwert und größer als ein zweiter Schwellenwert, der kleiner als der erste Schwellenwert ist, als eine leichte Richtungsänderungsbewegung zu bestimmen, wobei die mehreren Peaks gleich nach der durch die Richtungsänderungsbewegungs-Bestimmungseinheit (16) bestimmten Richtungsänderungsbewegung extrahiert werden, wobei
   die Merkmalsextraktionseinheit dazu ausgelegt ist, unter Verwendung der jeweiligen Daten des ersten Bewegungssensors (2) und des zweiten Bewegungssensors (3) ein Merkmal der durch die Bestimmungseinheit für eine leichte Richtungsänderungsbewegung bestimmten leichten Richtungsänderungsbewegung zu extrahieren.

4. Informationsverarbeitungssystem (9) nach einem der vorhergehenden Ansprüche, ferner aufweisend eine Gehabschnitts-Bestimmungseinheit, die dazu ausgelegt ist, Peaks in den ersten Daten und den zweiten Daten, wobei ein Rotationsbetrag in einer Vorwärtsrichtung, der durch einen Peak unter den Peaks anzeigt wird, gleich oder größer als ein dritter Schwellenwert ist, in einem Abschnitt von Beendigung der Richtungsänderungsbewegung bis zu Beginn einer nächsten Richtungsänderungsbewegung, als Bewegungskandidaten eines Gehens zu extrahieren, und einen Peak zu bestimmen, wobei der durch den Peak angezeigte Rotationsbetrag kleiner als der dritte Schwellenwert ist und gleich oder größer als ein vierter Schwellenwert, der kleiner als der dritte Schwellenwert ist, wobei der Peak vor und nach den extrahierten Bewegungskandidaten des Gehens als Beginn oder Ende eines Gehabschnitts, der durch die extrahierten Peaks angezeigt wird, erscheint.

5. Informationsverarbeitungssystem (9) nach Anspruch 4, wobei die Gehabschnitts-Bestimmungseinheit dazu ausgelegt ist, in dem Gehabschnitt einen Abschnitt, in dem eine Verteilung von aufeinanderfolgenden Zeitintervallen einer vorbestimmten Anzahl größer als ein Schwellenwert ist, als Übergangsabschnitt, der einen instabilen Gehabschnitt anzeigt, zu bestimmen.

6. Informationsverarbeitungssystem (9) nach Anspruch 5, wobei die Merkmalsextraktionseinheit dazu ausgelegt ist, unter Verwendung der jeweiligen Daten des ersten Bewegungssensors (2) und des zweiten Bewegungssensors (3) ein Merkmal des Übergangsabschnitts zu extrahieren.

7. Informationsverarbeitungssystem (9) nach einem der vorhergehenden Ansprüche, wobei der erste Bewegungssensor (2) und der zweite Bewegungssensor (3) jeweils einen Beschleunigungssensor umfassen.

8. Informationsverarbeitungssystem (9) nach einem der vorhergehenden Ansprüche, wobei die Informationsverarbeitungseinheit (1) durch einen von dem ersten Bewegungssensor (2) und dem zweiten Bewegungssensor (3) implementiert ist.

9. Informationsverarbeitungsverfahren, bei dem ein Computer (200) die Verarbeitung ausführt von:

Extrahieren, in einem System, in dem ein erster Bewegungssensor für ein linkes Bein eines Benutzers verwendet wird und ein zweiter Bewegungssensor für ein rechtes Bein des Benutzers verwendet wird, wobei der erste Bewegungssensor und der zweite Bewegungssensor jeweils einen Gyrosensor umfassen, der dazu ausgelegt ist, Daten einer Winkelgeschwindigkeit um drei senkrecht zueinander stehende Achsen zu erfassen, von mehreren Peaks in ersten Daten unter von dem ersten Bewegungssensor erfassten Daten und in zweiten Daten unter von dem zweiten Bewegungssensor erfassten Daten als Bewegungskandidaten eines Richtungswechsels, wobei die Peaks einen Rotationsbetrag des linken Beins bzw. des rechten Beins in einer Richtung quer zu einer Laufrichtung des Benutzers anzeigen, wobei der durch die Peaks angezeigte Rotationsbetrag größer als ein erster Schwellenwert ist, von einem Zeitpunkt an, zu dem ein Fuß vom Boden abhebt, bis zu einem Zeitpunkt der Landung, wobei, wenn mehr als einer der Peaks in den ersten Daten oder den zweiten Daten nacheinander erscheint, bei demselben Bein, in einer vorbestimmten Zeit, der mehr als eine der Peaks als ein einzelner Bewegungskandidat eines Schritts extrahiert wird;
Bestimmen einer durch die extrahierten Bewegungskandidaten angezeigten Bewegung als eine Richtungsänderungsbewegung, basierend darauf, ob die ersten Daten und die zweiten Daten anzeigen, dass ein Bewegungskandidat des linken Beins und ein Bewegungskandidat des rechten Beins sich abwechseln, zeitlich darin zu erscheinen, und basierend darauf, ob die Vorzeichen der Rotationsbeträge der abwechselnden Bewegungskandidaten gleich sind, aufweisend Bestimmen der durch die extrahierten Bewegungskandidaten angezeigten Bewegung als die Richtungsänderungsbewegung, wenn die Vorzeichen der Rotationsbeträge der abwechselnden Bewegungskandidaten gleich sind und wenn die ersten Daten und die zweiten Daten anzeigen, dass der Bewegungskandidat des linken Beins und der Bewegungskandidat des rechten Beins sich abwechseln, zeitlich darin zu erscheinen; und
Extrahieren eines Merkmals der bei der Bestimmung bestimmten Richtungsänderungsbewegung unter Verwendung der von dem ersten Bewegungssensor und von dem zweiten Bewegungssensor erfassten Daten.

10. Programm, das, wenn es von einem Computer ausgeführt wird, den Computer veranlasst, das Verfahren nach Anspruch 9 auszuführen.

**Revendications**

1. Système de traitement d'informations (9) comprenant :

un premier capteur de mouvement (2) qui est utilisé pour une jambe gauche d'un utilisateur ;
un second capteur de mouvement (3) qui est utilisé pour une jambe droite de l'utilisateur ;
dans lequel le premier capteur de mouvement (2) et le second capteur de mouvement (3) comprennent chacun un capteur gyroscopique configuré de manière à acquérir des données d'une vitesse angulaire autour de trois axes qui sont perpendiculaires les uns aux autres ; et

une unité de traitement d'informations (1) configurée de manière à traiter les données acquises par le premier capteur de mouvement et le second capteur de mouvement, dans lequel l'unité de traitement d'informations (1) inclut :

une unité d'extraction de candidats de mouvement (12) configurée de manière à extraire une pluralité de pics dans des premières données parmi les données acquises à partir du premier capteur de mouvement (2), et dans des secondes données parmi les données acquises à partir du second capteur de mouvement (3), en tant que des candidats de mouvement d'un changement de direction, dans lequel les pics indiquent une quantité de rotation de la jambe gauche ou de la jambe droite, respectivement, dans une direction transversale à une direction de marche de l'utilisateur, dans lequel la quantité de rotation de la jambe gauche ou de la jambe droite indiquée par les pics est supérieure à un premier seuil depuis un instant où un pied quitte le sol jusqu'à un instant d'atterrissage, dans lequel, lorsque plus d'un desdits pics apparaît successivement dans les premières données ou les secondes données au cours d'un temps prédéterminé sur la même jambe, l'unité est configurée de manière à extraire ledit plus d'un desdits pics en tant qu'un candidat de mouvement unique d'un pas ;

une unité de détermination de mouvement de changement de direction (16) configurée de manière à déterminer un mouvement indiqué par les candidats de mouvement extraits par l'unité d'extraction de candidats de mouvement (12), en tant qu'un mouvement de changement de direction, selon que les premières données et les secondes données indiquent qu'un candidat de mouvement de la jambe gauche et qu'un candidat de mouvement de la jambe droite alternent en y apparaissant temporellement, et selon que des signes des quantités de rotation des candidats de mouvement alternés sont les mêmes, dans lequel l'unité de détermination de mouvement de changement de direction (16) est configurée de manière à déterminer le mouvement indiqué par les candidats de mouvement extraits par l'unité d'extraction de candidats de mouvement (12), en tant que le mouvement de changement de direction lorsque les signes des quantités de rotation des candidats de mouvement alternés sont les mêmes, et lorsque les premières données et les secondes données indiquent que le candidat de mouvement de la jambe gauche et que le candidat de mouvement de la jambe droite alternent en y apparaissant temporellement ; et

une unité d'extraction de caractéristiques (17) configurée de manière à extraire une caractéristique du mouvement de changement de direction qui est déterminé par l'unité de détermination de mouvement de changement de direction (16), en utilisant les données acquises par le premier capteur de mouvement (2) et par le second capteur de mouvement (3).

2. Système de traitement d'informations (9) selon la revendication 1, dans lequel l'unité d'extraction de caractéristiques est configurée de manière à extraire des informations qui incluent au moins l'une parmi une quantité de rotation dans la direction gauche-droite, une vitesse de rotation, une durée de rotation, et une quantité d'avancement vers une direction vers l'avant, en tant que la caractéristique du mouvement de changement de direction.

3. Système de traitement d'informations (9) selon la revendication 1 ou 2, incluant en outre :

une unité de détermination de léger mouvement de changement de direction (16) configurée de manière à déterminer un mouvement indiqué par une pluralité de pics, dans lequel une quantité de rotation dans la direction gauche-droite d'un pic parmi les pics est inférieure audit premier seuil et est supérieure à un deuxième seuil qui est inférieur au premier seuil, en tant qu'un léger mouvement de changement de direction, la pluralité de pics étant extraite juste après le mouvement de changement de direction déterminé par l'unité de détermination de mouvement de changement de direction (16), dans lequel

l'unité d'extraction de caractéristiques est configurée de manière à extraire une caractéristique du léger mouvement de changement de direction déterminé par l'unité de détermination de léger mouvement de changement de direction, en utilisant les données respectives du premier capteur de mouvement (2) et du second capteur de mouvement (3).

4. Système de traitement d'informations (9) selon l'une quelconque des revendications précédentes, incluant en outre une unité de détermination de section de démarche configurée de manière à extraire des pics dans les premières données et les secondes données, dans lequel une quantité de rotation dans une direction vers l'avant indiquée par un pic parmi les pics est égale ou supérieure à un troisième seuil dans une section allant de la fin du mouvement de changement de direction jusqu'au début d'un mouvement de changement de direction suivant, en tant que des candidats de mouvement d'une démarche, et à déterminer un pic, dans lequel la quantité de rotation indiquée par le pic est inférieure audit troisième seuil et égale ou supérieure à un quatrième seuil qui est inférieur au troisième seuil, le pic apparaissant avant et après les candidats de mouvement extraits de la démarche, en tant que début ou fin d'une section de démarche indiquée par les pics extraits.

5. Système de traitement d'informations (9) selon la revendication 4, dans lequel l'unité de détermination de section de démarche est configurée de manière à déterminer, dans la section de démarche, une section dans laquelle une répartition d'intervalles de temps séquentiels d'un nombre prédéterminé est supérieure à un seuil, en tant qu'une section de démarche de transition qui indique une section de démarche instable.

6. Système de traitement d'informations (9) selon la revendication 5, dans lequel l'unité d'extraction de caractéristiques est configurée de manière à extraire une caractéristique de la section de démarche de transition en utilisant les données respectives du premier capteur de mouvement (2) et du second capteur de mouvement (3).

7. Système de traitement d'informations (9) selon l'une quelconque des revendications précédentes, dans lequel le premier capteur de mouvement (2) et le second capteur de mouvement (3) comprennent chacun un capteur d'accélération.

8. Système de traitement d'informations (9) selon l'une quelconque des revendications précédentes, dans lequel l'unité de traitement d'informations (1) est mise en œuvre par l'un quelconque du premier capteur de mouvement (2) et du second capteur de mouvement (3).

9. Procédé de traitement d'informations dans lequel un ordinateur (200) exécute un traitement consistant à :

extraire, dans un système dans lequel un premier capteur de mouvement est utilisé pour une jambe gauche d'un utilisateur, et un second capteur de mouvement est utilisé pour une jambe droite de l'utilisateur, dans lequel le premier capteur de mouvement et le second capteur de mouvement comprenant chacun un capteur gyroscopique configuré de manière à acquérir des données d'une vitesse angulaire autour de trois axes qui sont perpendiculaires les uns aux autres, une pluralité de pics dans des premières données parmi des données acquises à partir du premier capteur de mouvement, et dans des secondes données parmi les données acquises à partir du second capteur de mouvement, en tant que des candidats de mouvement d'un changement de direction, dans lequel les pics indiquent une quantité de rotation de la jambe gauche ou de la jambe droite, respectivement, dans une direction transversale à une direction de marche de l'utilisateur, dans lequel la quantité de rotation indiquée par les pics est supérieure à un premier seuil depuis un instant où un pied quitte le sol jusqu'à un instant d'atterrissage, dans lequel, lorsque plus d'un desdits pics apparaît successivement dans les premières données ou les secondes données au cours d'un temps prédéterminé sur la même jambe, extraire ledit plus d'un desdits pics en tant qu'un candidat de mouvement unique d'un pas ;
déterminer un mouvement indiqué par les candidats de mouvement extraits, en tant qu'un mouvement de changement de direction, selon que les premières données et les secondes données indiquent qu'un candidat de mouvement de la jambe gauche et qu'un candidat de mouvement de la jambe droite alternent en y apparaissant temporellement, et selon que des signes des quantités de rotation des candidats de mouvement alternés sont les mêmes, y compris déterminer le mouvement indiqué par les candidats de mouvement extraits, en tant que le mouvement de changement de direction lorsque les signes des quantités de rotation des candidats de mouvement alternés sont les mêmes, et lorsque les premières données et les secondes données indiquent que le candidat de mouvement de la jambe gauche et que le candidat de mouvement de la jambe droite alternent en y apparaissant temporellement ; et
extraire une caractéristique du mouvement de changement de direction qui est déterminé à l'étape de détermination, en utilisant les données acquises par le premier capteur de mouvement et par le second capteur de mouvement.

10. Programme qui, lorsqu'il est exécuté par un ordinateur, amène l'ordinateur à mettre en œuvre le procédé selon la revendication 9.

# FIG.1

INFORMATION
PROCESSING SYSTEM
9

INFORMATION PROCESSING APPARATUS    1

SENSOR DEVICE    2

| DATA ACQUIRING UNIT    21 |
| DATA TRANSMITTING UNIT    22 |

SENSOR DEVICE    3

| DATA ACQUIRING UNIT    31 |
| DATA TRANSMITTING UNIT    32 |

| DATA ACQUIRING UNIT    11 | → | MOTION-CANDIDATE EXTRACTING UNIT    12 | → | LEFT-RIGHT-SYMMETRY EXTRACTING UNIT    13 | → | TIME-FEATURE EXTRACTING UNIT    14 | → | PHASE-FEATURE EXTRACTING UNIT    15 |

| DIRECTION-CHANGE-SECTION DETERMINING UNIT    16 | → | DIRECTION-CHANGE-FEATURE EXTRACTING UNIT    17 | → | BEFORE-AND-AFTER-SECTION IDENTIFYING UNIT    18 | → | GAIT-SECTION DETERMINING UNIT    19 | → | GAIT-FEATURE EXTRACTING UNIT    20 |

| DATA STORAGE UNIT    23 |

| FEATURE-AMOUNT STORAGE UNIT    21 |

| FEATURE-AMOUNT VISUALIZING UNIT    22 |

EP 3 396 319 B1

# FIG.2A

<WHEN VIEWED FROM RIGHT SIDE>

<WHEN VIEWED FROM TOP>

TRAVELING DIRECTION

Z-AXIS

Y-AXIS

FORWARD DIRECTION OF ROTATION ABOUT X-AXIS (FORE-AND-AFT ROTATION)

GROUND

SENSOR DEVICE

GROUND

X-AXIS

FORWARD DIRECTION OF ROTATION ABOUT Z-AXIS (LEFT-RIGHT ROTATION)

TRAVELING DIRECTION

Y-AXIS

# FIG.2B

DATA COMMUNICATION

3
SENSOR DEVICE

2
SENSOR DEVICE

1
INFORMATION PROCESSING
APPARATUS

# FIG.3

# FIG.4

[ROTATE IN TRAVERSE DIRECTION]

BEFORE
FOOT-OFF

AFTER FOOT
STRIKE

100 ms

p11
p12

p10

# FIG.5

DIRECTION
CHANGE ENDS

NEXT DIRECTION
CHANGE STARTS

p22  p24  p26

THRESHOLD
R1

SENSOR DEVICE 2
(LEFT LEG GYRO)

p21  p23  p25  p27

SENSOR DEVICE 3
(RIGHT LEG GYRO)

06:38:08          06:38:13

DIRECTION CHANGE
SECTION

p20
FIRST ONE STEP

p30
LAST ONE STEP

SENSOR DEVICE 2
(LEFT LEG GYRO)

THRESHOLD
R2

SENSOR DEVICE 3
(RIGHT LEG GYRO)

06:38:08          06:38:13

GAIT SECTION
CANDIDATE

GAIT SECTION

# FIG.6

```
          ┌─────────────┐
          │    START    │
          └─────────────┘
                 │
                 ▼
  ┌──────────────────────────────┐
  │ ACQUIRE GYRO SENSOR DATA OF   │ ～S11
  │         BOTH LEGS             │
  └──────────────────────────────┘
                 │
                 ▼
  ┌──────────────────────────────┐
  │  EXTRACT DIRECTION CHANGE     │ ～S12
  │          SECTION              │
  └──────────────────────────────┘
                 │
                 ▼
  ┌──────────────────────────────┐
  │  EXTRACT FEATURE AMOUNT OF    │ ～S13
  │      DIRECTION CHANGE         │
  └──────────────────────────────┘
                 │
                 ▼
  ┌──────────────────────────────┐
  │   IDENTIFY BEFORE-AND-AFTER   │
  │ SECTION OF DIRECTION CHANGE   │ ～S14
  │          SECTION              │
  └──────────────────────────────┘
                 │
                 ▼
  ┌──────────────────────────────┐
  │   EXTRACT TRANSITION GAIT     │ ～S15
  │          SECTION              │
  └──────────────────────────────┘
                 │
                 ▼
  ┌──────────────────────────────┐
  │  EXTRACT FEATURE AMOUNT OF    │ ～S16
  │       TRANSITION GAIT         │
  └──────────────────────────────┘
                 │
                 ▼
  ┌──────────────────────────────┐
  │  STORE FEATURE AMOUNT OF      │
  │  DIRECTION CHANGE AND         │ ～S17
  │    TRANSITION GAIT IN DB      │
  └──────────────────────────────┘
                 │
                 ▼
  ┌──────────────────────────────┐
  │   VISUALIZE FEATURE AMOUNT    │ ～S18
  └──────────────────────────────┘
                 │
                 ▼
          ┌─────────────┐
          │     END     │
          └─────────────┘
```

# FIG.7A

```
START
```

↓ S21

EXTRACT MOTION CANDIDATE POINT
FROM GYRO DATA IN TRAVERSE
DIRECTION OF LEFT LEG

↓ S22

EXTRACT MOTION CANDIDATE POINT
FROM GYRO DATA IN TRAVERSE
DIRECTION OF RIGHT LEG

↓ S23

CHUNKING OF MULTIPLEX PEAKS

↓ S24

i=1

↓ S25

CALCULATE ROTATION AMOUNT OF i-TH
MOTION CANDIDATE

↓ S26

EXCLUDE FROM CANDIDATE POINT
WHEN ROTATION AMOUNT IS EQUAL TO
OR SMALLER THAN THRESHOLD

↓ S27

i<N (N: TOTAL NUMBER OF MOTION CANDIDATE POINTS)?

YES → S28

i=i+1

NO → (1)

# FIG.7B

```
                    ( 1 )
                      │
                      ▼
┌──────────────────────────────────────┐  ─S29
│                 i=1                    │
└──────────────────────────────────────┘
                      │
                      ▼
┌──────────────────────────────────────┐  ─S30
│  ACQUIRE TIME INFORMATION OF i-TH      │
│  AND i+1-TH MOTION CANDIDATE POINTS    │
│  AND BELONGING LEG SIDE INFORMATION    │
└──────────────────────────────────────┘
                      │
                      ▼
┌──────────────────────────────────────┐  ─S31
│  EXTRACT LEFT RIGHT SYMMETRY OF        │
│  TWO CANDIDATE POINTS                  │
└──────────────────────────────────────┘
                      │
                      ▼
┌──────────────────────────────────────┐  ─S32
│  EXTRACT TIME FEATURE OF TWO           │
│  CANDIDATE POINTS                      │
└──────────────────────────────────────┘
                      │
                      ▼
┌──────────────────────────────────────┐  ─S33
│  EXTRACT PHASE FEATURE OF TWO          │
│  CANDIDATE POINTS                      │
└──────────────────────────────────────┘
                      │
                      ▼
               ◇─────────────◇  ─S34
        YES   ╱  i<m (M: TOTAL ╲
       ┌──────   NUMBER OF MOTION CANDIDATE
       │      ╲    POINTS)?   ╱
       ▼       ◇─────────────◇
┌─────────────┐ ─S35         │ NO
│   i=i+1      │             ▼
└─────────────┘            ( 2 )
```

# FIG.7C

②

S36

ACQUIRE DETERMINATION PARAMETERS

S37

DETERMINE SECTION IN WHICH
CANDIDATE POINTS OF LEFT LEG AND
RIGHT LEG APPEAR "ALTERNATELY",
"SUCCESSIVELY", AND "IN SAME PHASE"
AS DIRECTION CHANGE SECTION

END

# FIG.8

```
                            ┌─────────────┐
                            │    START    │
                            └──────┬──────┘
                                   │              ┌S41
                    ┌──────────────▼──────────────┐
                    │             j=1             │
                    └──────────────┬──────────────┘
                                   │              ┌S42
                    ┌──────────────▼──────────────┐
                    │             i=1             │
                    └──────────────┬──────────────┘
                                   │              ┌S43
                    ┌──────────────▼──────────────┐
                    │ ACQUIRE TIME INFORMATION OF │
                    │ i-TH MOTION POINT IN j-TH   │
                    │ DIRECTION CHANGE SECTION    │
                    └──────────────┬──────────────┘
                                   │              ┌S44
                    ┌──────────────▼──────────────┐
                    │ (a) EXTRACT ROTATION AMOUNT │
                    │   OF SUBJECT MOTION POINT   │
                    └──────────────┬──────────────┘
                                   │              ┌S45
                    ┌──────────────▼──────────────┐
                    │ (b) EXTRACT ROTATION SPEED  │
                    │   OF SUBJECT MOTION POINT   │
                    └──────────────┬──────────────┘
                                   │              ┌S46
                    ┌──────────────▼──────────────┐
                    │ (c) EXTRACT ROTATION        │
                    │ DURATION OF SUBJECT MOTION  │
                    │ POINT                       │
                    └──────────────┬──────────────┘
                                   │              ┌S47
                    ┌──────────────▼──────────────┐
                    │ (d) EXTRACT ADVANCEMENT     │
                    │ AMOUNT OF SUBJECT MOTION    │
                    │ POINT                       │
                    └──────────────┬──────────────┘
                                   │              ┌S48
              YES          ◇───────▼───────◇
        ┌S49  ┌────────────  i<M (M: TOTAL NUMBER
   ┌────▼────┐│              OF MOTION CANDIDATE
   │  i=i+1  ││              POINTS)?
   └─────────┘◇──────────────◇
                                   │ NO
```

S50 — CALCULATE AVERAGE, MAXIMUM VALUE, MINIMUM VALUE, DISTRIBUTION, AND LEFT-RIGHT RATIO OF FEATURE AMOUNTS (a) TO (d)

S51 — EXTRACT FEATURE AMOUNTS (a) TO (d) OF DIRECTION CHANGE START MOTION AND DIRECTION CHANGE END MOTION

S52 — CALCULATE NUMBER OF TOTAL MOTION POINTS, SECTION WIDTH, TOTAL ROTATION AMOUNT, TOTAL ADVANCEMENT AMOUNT, ROTATION AMOUNT/ADVANCEMENT AMOUNT RATIO, WHETHER STILL STATE SECTION IS PRESENT

S53 — j<L (L: TOTAL NUMBER OF DIRECTION CHANGE SECTIONS)?

YES — S54 — j=j+1

NO — END

# FIG.9A

START

S61

EXTRACT MOTION CANDIDATE POINTS FROM GYRO DATA IN FORE-AND-AFT DIRECTION OF LEFT LEG

S62

EXTRACT MOTION CANDIDATE POINTS FROM GYRO DATA IN FORE-AND-AFT DIRECTION OF RIGHT LEG

S63

p=1

S64

CALCULATE GAIT SPEED AMOUNT OF p-TH MOTION CANDIDATE POINT

S65

EXCLUDE FROM CANDIDATE POINT WHEN GAIT SPEED AMOUNT IS EQUAL TO OR SMALLER THAN THRESHOLD

S66

p<P (P: TOTAL NUMBER OF MOTION CANDIDATE POINTS)?

YES

S67

p=p+1

NO

4

# FIG.9B

④

S68

q=1

S69

ACQUIRE TIME INFORMATION AND
BELONGING LEG SIDE INFORMATION OF
Q-TH AND Q+1-TH MOTION CANDIDATE
POINTS

S70

EXTRACT LEFT-RIGHT SYMMETRY OF
TWO CANDIDATE POINTS

S71

EXTRACT TIME FEATURE OF TWO
CANDIDATE POINTS

S72

q<Q (Q: TOTAL
NUMBER OF MOTION CANDIDATE
POINTS)?

YES

NO

S73

q=q+1

⑤

# FIG.9C

⑤

**S74**

ACQUIRE DETERMINATION PARAMETERS

**S75**

DETERMINE SECTION IN WHICH CANDIDATE POINTS OF LEFT LEG AND RIGHT LEG APPEAR "ALTERNATELY" AND "SUCCESSIVELY" AS GAIT SECTION

**S76**

EXTRACT TIME INTERVAL BETWEEN MOTION POINTS IN DETERMINED GAIT SECTION

**S77**

i=1

**S78**

ACQUIRE TIME INTERVAL OF i-TH TO (i+n-1)-TH MOTION POINTS

**S79**

CALCULATE DISTRIBUTION OF TIME INTERVALS OF n PIECES OF MOTION CANDIDATES

**S80**

IS DISTRIBUTION SMALLER THAN THRESHOLD (CONVERGENCE DETERMINATION)?

NO

**S81**

i=i+1

YES

**S82**

IDENTIFY SECTION BETWEEN TIME AT WHICH FIRST GAIT STEP STARTS UNTIL TIME AT WHICH (i-1)-TH GAIT STEP ENDS AS TRANSITION GAIT SECTION

END

FIG.10

START

r=1 ～S91

q=1 ～S92

ACQUIRE TIME INFORMATION OF q-TH
MOTION POINT IN r-TH TRANSITION GAIT
SECTION ～S93

(v) EXTRACT STEP LENGTH OF SUBJECT
MOTION POINT ～S94

(w) EXTRACT GAIT SPEED OF SUBJECT
MOTION POINT ～S95

(x) EXTRACT SWING PHASE DURATION OF
SUBJECT MOTION POINT ～S96

(y) EXTRACT FOOT LIFT AMOUNT OF
SUBJECT MOTION POINT ～S97

(z) EXTRACT LEFT-RIGHT MOVEMENT
AMOUNT OF SUBJECT MOTION POINT ～S98

S99
q<Q (Q: TOTAL
NUMBER OF MOTION CANDIDATE
POINTS)? ——YES

NO

S100
q=q+1

CALCULATE AVERAGE VALUE, MAXIMUM
VALUE, MINIMUM VALUE, DISTRIBUTION,
LEFT-RIGHT RATIO OF FEATURE
AMOUNTS (v) TO (z) ～S101

EXTRACT FEATURE AMOUNTS (v) TO (z)
OF FIRST STEP AND SECOND STEP ～S102

EXTRACT TIME INTERVAL AND LEG
MOVEMENT AMOUNT IN SECTION FROM
TERMINATION OF DIRECTION CHANGE
AND START OF TRANSITION GAIT ～S103

S104
r<R (R: TOTAL NUMBER
OF GAIT SECTIONS)? ——YES

S105
r=r+1

NO

END

# FIG.11A

| TIME | ··· | 21:06:51.800 | 21:06:51.820 | ··· | 21:07:14.400 | ··· |
|---|---|---|---|---|---|---|
| TRAVERSE DIRECTION GYRO (deg/s) | ··· | 0.0 | 0.3 | ··· | 0.8 | ··· |

# FIG.11B

| PEAK NUMBER | OCCURRENCE TIME |
|---|---|
| 1 | 21:06:55.640 |
| 2 | 21:06:56.840 |
| 3 | 21:06:58.240 |
| 4 | 21:06:58.940 |

## FIG.11C

| PEAK NUMBER | OCCURRENCE TIME |
|---|---|
| 1 | 21:06:55.640 |
| 2 | 21:06:56.840 |
| 3 | 21:06:58.240 |
| 4 | 21:06:58.940 |

⇒

| PEAK NUMBER | OCCURRENCE TIME |
|---|---|
| 1 | 21:06:55.640 |
| 2 | 21:06:56.840 |
| 3 | 21:06:58.590 |

CHUNKING

21:06:52　21:06:57　21:07:02　21:07:07

# FIG.11D

EP 3 396 319 B1

# FIG.11E

<LIST OF CANDIDATE POINTS>

| CANDIDATE POINT NUMBER | OCCUR-RENCE TIME | START POSITION | END POSITION | LEG SIDE |
|---|---|---|---|---|
| 1 | 21:06:55. 640 | 21:06:55. 440 | 21:06:55. 940 | RIGHT |
| 2 | 21:06:55. 980 | 21:06:55. 920 | 21:06:56. 180 | LEFT |
| ... | ... | ... | ... | ... |
| 6 | 21:06:57. 560 | 21:06:57. 220 | 21:06:57. 980 | LEFT |
| 7 | 21:06:58. 940 | 21:06:58. 900 | 21:06:59. 200 | RIGHT |

EP 3 396 319 B1

# FIG.12A

FEATURE AMOUNT CALCULATION RESULT

<LIST OF CANDIDATE POINTS>

| CANDIDATE POINT NUMBER | OCCURRENCE TIME | START POSITION | END POSITION | LEG SIDE | FEATURE AMOUNT C1 | FEATURE AMOUNT C2 | FEATURE AMOUNT C3 |
|---|---|---|---|---|---|---|---|
| 1 | 21:06:55.640 | 21:06:55.440 | 21:06:55.940 | RIGHT | 0 | 0.34 | 1 |
| 2 | 21:06:55.980 | 21:06:55.920 | 21:06:56.180 | LEFT | 0 | 0.30 | 1 |
| ... | ... | ... | ... | ... | ... | ... | ... |
| 6 | 21:06:57.240 | 21:06:56.920 | 21:06:57.480 | LEFT | 0 | 1.7 | 1 |
| 7 | 21:06:58.940 | 21:06:58.900 | 21:06:59.200 | RIGHT | 1 | 12.2 | 1 |

EP 3 396 319 B1

# FIG.12B

S0

| CANDIDATE POINT NUMBER | FEATURE AMOUNT C1 | FEATURE AMOUNT C2 | FEATURE AMOUNT C3 | STATE | DIRECTION CHANGE MOTION |
|---|---|---|---|---|---|
| 1 | 0 | 0.34 | 1 | STATE 1 | ○ |
| 2 | 0 | 0.30 | 1 | STATE 2 | ○ |
| ... | ... | ... | ... | ... | ... |
| 6 | 0 | 1.7 | 1 | STATE 3 | ○ |
| 7 | 1 | 12.2 | 1 | STATE 4 | × |

STATE 2
MOTION DURING DIRECTION CHANGE

STATE 1
ROTATION START MOTION

STATE 3
ROTATION END MOTION

OTHER MOTION

STATE 4

EP 3 396 319 B1

# FIG.12C

STATE $S^{(i+1)}$ OF NEXT CANDIDATE POINT

| | | STATE 1 | STATE 2 | STATE 3 | STATE 4 | |
|---|---|---|---|---|---|---|
| STATE $S^{(i)}$ OF CURRENT CANDIDATE POINT | STATE 1 | 0 | 1 | 0 | 0 | |
| | STATE 2 | 0 | 0.5 | 0.5 | 0 | |
| | STATE 3 | 0.5 | 0 | 0 | 0.5 | d1 |
| | STATE 4 | 0.5 | 0 | 0 | 0.5 | |

# FIG.12D

<GAUSSIAN MODEL OF FEATURE AMOUNT C1>

$p(x_1^{(i)}|S=\text{STATE 2})$

PROBABILITY DENSITY

$\sigma_1$

0     1     FEATURE AMOUNT C1 (LEFT-RIGHT SYMMETRY)

$N(x_1^{(i)}|\mu_1=0,\ \sigma_1=0.01,\ S=\text{STATE 2})$

<GAUSSIAN MODEL OF FEATURE AMOUNT C2>

$p(x_2^{(i)}|S=\text{STATE 2})$

PROBABILITY DENSITY

$\sigma_2$

0   0.5   1    FEATURE AMOUNT C2 (TIME INTERVAL)

$N(x_2^{(i)}|\mu_2=0.5,\ \sigma_2=1,\ S=\text{STATE 2})$

<GAUSSIAN MODEL OF FEATURE AMOUNT C3>

$p(x_3^{(i)}|S=\text{STATE 2})$

PROBABILITY DENSITY

$\sigma_3$

0     1     FEATURE AMOUNT C3 (ROTATION DIRECTION MATCHING)

$N(x_3^{(i)}|\mu_3=1,\ \sigma_3=0.01,\ S=\text{STATE 2})$

# FIG.13A

| MOTION POINT NUMBER | START POSITION | END POSITION | DIRECTION CHANGE MOTION |
|:---:|:---:|:---:|:---:|
| 1 | 21:06:55.440 | 21:06:55.940 | ○ |
| 2 | 21:06:55.920 | 21:06:56.180 | ○ |
| … | … | … | … |
| 6 | 21:06:56.920 | 21:06:57.480 | ○ |

# FIG.13B

MOTION POINT NUMBER

(c) ROTATION DURATION

(a)
ROTATION
AMOUNT

(b) ROTATION
SPEED

(d) FORWARD
MOVEMENT
SPEED

(e) TIME INTERVAL

12:06:46                    12:06:51

| MOTION POINT NUMBER | START POSI-TION | END POSI-TION | ROTA-TION AMOUNT | ROTA-TION DURA-TION | ROTA-TION SPEED | TIME INTER-VAL | FORWARD MOVEMENT SPEED |
|---|---|---|---|---|---|---|---|
| 1 | 06:55.4 | 06:55.9 | 0.45 | 0.24 | 0.8 | 0.22 | 0.21 |
| 2 | 06:55.9 | 06:56.2 | 0.64 | 0.33 | 1.2 | 0.12 | 0.11 |
| … | … | … | … | … | … | … | … |
| 6 | 06:56.9 | 06:57.5 | 0.56 | 0.38 | 1.01 | 0.1 | 0.45 |

49

# FIG.13C

| STATISTIC | ROTATION AMOUNT | ROTATION DURATION | ROTATION SPEED | TIME INTERVAL | FORWARD MOVEMENT SPEED |
|---|---|---|---|---|---|
| AVERAGE | 0.55 | 0.29 | 1.15 | 0.18 | 0.27 |
| MAXIMUM VALUE | 0.64 | 0.39 | 1.47 | 0.22 | 0.45 |
| MINIMUM VALUE | 0.45 | 0.26 | 0.80 | 0.1 | 0.11 |
| DISTRIBUTION | 0.03 | 0.09 | 0.21 | 0.005 | 0.03 |
| LEFT-RIGHT RATIO | 1.42 | 1.32 | 1.40 | 0.38 | 1.22 |
| DIRECTION CHANGE START MOTION | 0.45 | 0.24 | 0.80 | 0.22 | 0.21 |
| DIRECTION CHANGE END MOTION | 0.56 | 0.38 | 1.01 | 0.1 | 0.45 |

| NUMBER OF MOTION POINTS | SECTION WIDTH | TOTAL ROTATION AMOUNT | TOTAL ADVANCE-MENT AMOUNT | ROTATION AMOUNT/ ADVANCEMENT AMOUNT RATIO | PRESENCE OF STILL STATE SECTION |
|---|---|---|---|---|---|
| 6 | 2.04 | 6.91 | 3.45 | 2.01 | NO |

# FIG.14A

DIRECTION CHANGE SECTION

BEFORE-AND-AFTER SECTION OF DIRECTION CHANGE SECTION

DETECTED PEAK (=CANDIDATE POINT OF GAIT MOTION)

# FIG.14B

# FIG.14C

# FIG.14D

<TRANSITION GAIT SECTION
DETERMINATION OF FIRST STEP>

| MOTION POINT NUMBER | FEATURE AMOUNT (2) (TIME INTERVAL) | STABILITY | TRANSITION GAIT SECTION |
|---|---|---|---|
| 1 | 0.24 | × | ○ |
| 2 | 0.20 | D11 | |
| 3 | 0.08 | | |
| 4 | 0.07 | | |
| 5 | 0.04 | | |
| 6 | 0.05 | | |
| 7 | 0.07 | | |
| 8 | 0.05 | | |
| 9 | 0.06 | | |
| 10 | 9.3 | | |

D1

<TRANSITION GAIT SECTION
DETERMINATION OF THIRD STEP>        D31

| MOTION POINT NUMBER | FEATURE AMOUNT (2) (TIME INTERVAL) | STABILITY | TRANSITION GAIT SECTION |
|---|---|---|---|
| 1 | 0.24 | × | ○ |
| 2 | 0.20 | × | ○ |
| 3 | 0.08 | ○ | × |
| 4 | 0.07 | D21 | × |
| 5 | 0.04 | | × |
| 6 | 0.05 | | × |
| 7 | 0.07 | | × |
| 8 | 0.05 | | × |
| 9 | 0.06 | | × |
| 10 | 9.3 | | × |

D2

EP 3 396 319 B1

# FIG.15

| MOTION POINT NUMBER | TRANSITION GAIT SECTION | STEP LENGTH | SWING PHASE DURATION | GAIT SPEED | LEFT-RIGHT MOVE-MENT AMOUNT | FOOT LIFT UP AMOUNT |
|---|---|---|---|---|---|---|
| 1 | ○ | 2.31 | 0.85 | 0.5 | 0.06 | 0.1 |
| 2 | ○ | 3.45 | 0.69 | 0.41 | 0.09 | 0.18 |
| 3 | ✕ | -- | -- | -- | -- | -- |
| … | ✕ | -- | -- | -- | -- | -- |

# FIG.16

TIME INTERVAL T

12:06:46

12:06:51

DIRECTION
CHANGE
SECTION

GAIT SECTION
(INCLUDING
TRANSITION GAIT
SECTION)

# FIG.17

| DIREC-TION CHANGE SECTION NUMBER | FEATURE AMOUNT OF DIRECTION CHANGE SECTION | | | | | | FEATURE AMOUNT OF TRANSITION GAIT SECTION | | | | | |
|---|---|---|---|---|---|---|---|---|---|---|---|---|
| | START TIME | END TIME | NUMBER OF MOTIONS | SECTION WIDTH | TOTAL ROTA-TION AMOUNT | ... | GAIT RIGHT AFTER | START TIME | END TIME | TRANSI-TION GAIT SECTION WIDTH | NUMBER OF TRANSITION GAIT STEPS | ... |
| 33 | ... | ... | ... | ... | ... | ... | ... | ... | ... | ... | ... | ... |
| 34 | 21:06: 55.440 | 21:06: 57.480 | 6 | 2.04 | 6.91 | ... | YES | 21:06: 58.940 | 21:07: 02.080 | 1.71 | 2 | ... |
| ... | ... | ... | ... | ... | ... | ... | ... | ... | ... | ... | ... | ... |

# FIG.18

# FIG.19

EP 3 396 319 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014118767 A1
- JP 2009075036 A
- JP 10113343 A
- JP 2008027429 A
- JP 2009150711 A
- JP 2010223829 A

**Non-patent literature cited in the description**

- Automated detection of gait initiation and termination using wearable sensors. *Medical Engineering & Physics,* vol. 35 (12), 1713-1720